# EUROPEAN PATENT APPLICATION

(11) **EP 0 933 426 A1**
(43) Date of publication of application: **04.08.1999**
(21) Application number: 99106005.4
(22) Date of filing: 28.12.1990
(51) Int. Cl.: C12N 15/40, C07K 14/18, A61K 39/29, C12Q 1/70, G01N 33/569

(54) **Non-a, non-b hepatitis virus genomic cdna fragments and antigen polypeptides**

(30) Priority: 25.06.1990 JP 16746690; 31.08.1990 JP 23092190; 09.11.1990 JP 30560590
(62) Divisional of application: 90314371.7
(71) Applicant: The Research Foundation for Microbial Diseases of Osaka University, Suita-shi Osaka (JP)
(72) Inventor: Okayama, Hiroto, Minoo-shi, Osaka-fu (JP); Fuke, Isao, Takamatsu-shi, Kagawa-ken (JP); Mori, Chisato, Kanonji-shi, Kagawa-ken (JP); Takamizawa, Akihisa, Kanonji-shi, Kagawa-ken (JP); Yoshida, Iwao, Kanonji-shi, Kagawa-ken (JP)
(74) Representative: BROOKES & MARTIN

(57) **Abstract**

Disclosed are isolated non-A, non-B hepatitis virus genomic cDNA fragments of the entire region of the virus gene nucleotide sequence from the 1st to 9416th nucleotides shown in Fig. 2(1) through Fig. 2(16) hereof. The fragments are in particular a cDNA covering the sequence from the 333rd to the 422nd nucleotide, the coding region from the 333rd to 9362nd nucleotides. The cDNA fragments and antigen polypeptide expression products thereof are useful as a diagnostic reagent for non-A, non-B hepatitis. The antigen polypeptide is also useful as an active ingredient for a non-A, non-B hepatitis virus vaccine.

## Description

### Background of the Invention

### Field of the Invention

The present invention relates to a non-A, non-B hepatitis virus genomic cDNA and a non-A, non-B hepatitis virus antigen polypeptide. More particularly, the present invention is concerned with a non-A, non-B hepatitis virus genomic cDNA which is useful for producing a non-A, non-B hepatitis virus antigen polypeptide and with a non-A, non-B hepatitis antigen polypeptide which is an expression product thereof. The non-A, non-B hepatitis virus genomic cDNA of the present invention is also useful for genetically diagnosing non-A, non-B hepatitis. Further, the non-A, non-B hepatitis antigen polypeptide of the present invention is useful for producing a vaccine for non-A, non-B hepatitis, an immunoglobulin, a polyclonal or monoclonal antibody, an immunological diagnostic reagent, an agent for screening blood for transfusion and an agent for use in affinity chromatography for removing non-A, non-B hepatitis virus from blood for transfusion.

### Discussion of Related Art

### Definition of non-A, non-B hepatitis virus:

The viral hepatitis is a liver disease caused by the infection of a hepatitis virus. Heretofore, hepatitis A virus, hepatitis B virus and hepatitis D (delta) virus have been isolated and identified. The hepatitis D virus (delta-hepatitis virus) is a deficient virus which cannot multiply by itself and requires for its multiplication the co-presence of hepatitis B virus as a helper virus. Therefore, the hepatitis D virus is present only in a patient having hepatitis B. In 1974, it was reported that there were many patients having hepatitis caused by a factor other than the infection with either hepatitis A virus or hepatitis B virus. Such a hepatitis was named "non-A, non-B hepatitis", and researches on the non-A, non-B hepatitis virus have been made extensively and intensively throughout the world. Heretofore, it has been found that a plurality of types of non-A, non-B hepatitis viruses exist. Results of the researches up to now show that the non-A, non-B hepatitis virus is classified into two types according to the infection route, that is, an epidemic hepatitis virus, namely an enterically-transmitted non-A, non-B hepatitis virus, which is spread through water and food; and a blood transmitted non-A, non-B hepatitis virus which is spread through blood by transfusion, etc. Of the non-A, non-B hepatitis viruses, only an enterically-transmitted non-A, non-B hepatitis virus which spreads over the areas of Africa, India and Southeast Asia has been virologically identified, but the blood-transmitted non-A, non-B hepatitis virus has not yet been identified.

Hereinbelow, the blood-transmitted non-A, non-B hepatitis is often referred to simply as "NANB hepatitis", and the blood-transmitted non-A, non-B hepatitis virus is often referred to simply as "NANBV". Current situation of the studies on NANB hepatitis and problems:

With respect to the epidemiology, clinical examination, diagnosis, treatment and prevention of the NANB hepatitis, virological studies have been made in the world by the comparison of NANBV with the other hepatitis viruses, based on the knowledge of diagnostics, histopathology, immunology, molecular biology and the like ["Japan Medical Journal", No. 3320, pp.3-10, 1987; "Igaku-no Ayumi (Progress of medicine)", 151(13), pp.735-923, 1989; "Kan Tan Sui (Liver, Gallbladder, Pancreas)", 21(1), pp.5-113, 1990; "Jikken Igaku (Experimental Medicine)", 8(3), pp.201-233, 1990]. With respect to the NANB hepatitis, the following findings have been reported.
(1) Epidemiology: In Japan, according to the estimation by the Ministry of Health and Welfare, about 60 % of chronic hepatitis patients (namely about 720 thousand patients), about 40 % of hepatocirrhosis patients (namely about 100 thousand patients) and about 40 % of liver cancer patients (namely about 7 thousand patients) are patients having NANB hepatitis. Further, the mortality attributed to the above-mentioned NANB hepatitis reaches 16 thousand per year. In U.S.A., the number of post-transfusion hepatitis patients reaches 150 to 300 thousand per year and 90 % of the post-transfusion hepatitis patients are patients having NANB hepatitis. Further, it is considered that 1 to 6 % of the blood donors are NANBV carriers. Further, it is estimated that in the other countries also, the incidence of NANB hepatitis and the ratio of the NANBV carrier are equal to or higher than those in U.S.A. and Japan. Therefore, prevention, early diagnosis and early treatment of the NANB hepatitis are of global importance.
(2) Virology: The NANBV heretofore reported comprises an envelope and assumes a viral particle having a spherical shape of about 50 nm in diameter. The taxonomic observations suggest that the known NANBV is a virus similar to a togavirus or a flavivirus, or a virus of new type different from the togavirus or flavivirus. Further, the results of pathological observations of the cytoplasm of hepatocytes of a plurality of chimpanzees injected with serum of a patient having NANBV hepatitis show that the formation of a tubular structure occurs in the cytoplasm of a hepatocyte of some of the chimpanzees, but does not occur in the cytoplasm of a hepatocyte of the other chimpanzees, and that an intranuclear particle is formed in the cytoplasm of a hepatocyte of some of the chimpanzees. These results and the results of the epidemiological observations, tests on the presence or absence of the chloroform sensitivity and immunological diagnosis suggest that a plurality of types of NANBV-sexist (see, for example, "Science", Vol. 205, pp.197-200, 1979, "Journal of Infectious Disease", Vol. 148, pp.254-265, 1983, and "Biseibutsu" (Microorganism), Vol. 5, No. 5, pp.463-475, 1989). The amount of the NANBV present in the blood of a patient having NANB hepatitis is extremely small as compared to either the amount of a hepatitis A virus present in the feces of a patient having hepatitis A or the amount of a hepatitis B virus present in the blood of a patient having hepatitis B. For example, the amount of hepatitis B virus in the blood of the patient is 10⁸ to 10⁹ per ml in terms of Chimpanzee Infectious dose (CID), whereas the amount of NANBV in the blood of the patient is only 10⁴ to 10⁵ per ml in terms of CID (Bradley, D.W.: Research perspectives in post-transfusion non-A, non-B hepatitis, in "Infection, Immunity and Blood Transfusion", edited by Dodd, R.Y. & Barker, L.F., published by Alan R. Liss, Inc., New York (1985) pp.81-97). Further, it is known that except for human, there are no animals except chimpanzee that are sensitive to NANBV and that in the cytoplasm of the hepatocyte, a typical tubular structure is occasionally formed by NANBV infection. Since only chimpanzee can be used as an animal for experiment of the NANBV infection, a large number of chimpanzees are required to be used for the study of NANBV. However, the chimpanzee is not easily available and expensive. Therefore, the study of NANBV by, for example, experimental infection by NANBV, identification of NANBV and search for a useful marker for NANBV, is necessarily restricted and delayed. In order to solve these problems, various attempts have been made for the study of NANBV. For example, in an attempt, an NANBV genomic cDNA [(referred to as "hepatitis C virus (HCV)"] was cloned from blood plasma of chimpanzees suffering from NANB hepatitis (Science, Vol. 244, pp.359-362, 1989), and it was confirmed that the antigen (referred to as "C-100") obtained by expressing the cDNA exhibited an antigen-antibody reaction with the antibody in the blood of an NANB hepatitis patient (Science, Vol. 244, pp.362-364, 1989). Further, in another attempt, a chimpanzee was not used and an NANBV genomic cDNA was cloned from the blood plasma of NANB hepatitis patients, and it was confirmed that the antigen obtained by expressing the cDNA exhibited an antigen-antibody reaction with the antibody in the serum of an NANB hepatitis patient (Gastroenterologia Japonica, Vol. 24, pp.540-544 and pp.545-548, 1989).
(3) Clinical observations: Hepatitis is generally classified either into epidemic hepatitis and sporadic hepatitis according to the number and frequency of the occurrences of hepatitis, or into acute hepatitis, fulminant hepatitis, subacute hepatitis, persistent hepatitis and chronic hepatitis according to the severeness and stage of the hepatitis patients. The latent period of the NANB hepatitis is 2 to 26 weeks. The symptom of NANB hepatitis in the early stage is mild as compared to that of hepatitis B. For example, a patient having NANB hepatitis only becomes feverish and complains of languor. Further, 70 % of the patients have anicteric symptom. Therefore, the NANB hepatitis is frequently overlooked. However, the NANB hepatitis is very dangerous because the NANB hepatitis is likely to become chronic and, then, to progress to liver cirrhosis. Illustratively stated, 40 to 50 % of the patients having NANB hepatitis whose serum exhibits an increased aminotransferase activity develop chronic hepatitis. 10 to 20 % of the cases of chronic hepatitis suffer from liver cirrhosis. Further, 0.5 to 1 % of blood recipients per year becomes liver cirrhosis patients without subjective symptoms. More seriously, the liver cirrhosis may further progress to liver cancer or hepatoma. Therefore, for preventing biohazard caused by blood transfusion and bleeding, eradication of the NANB hepatitis is a matter of global importance from the viewpoint of public health.
(4) Diagnosis: As mentioned above, the NANBV (blood-transmitted type) has not yet been identified and a viral marker, such as an NANBV antigen, which is useful for the diagnosis of NANB hepatitis has not been known. Therefore, diagnosis of NANB hepatitis has been conducted by examining the titer of the antibody in serum of a patient, which is specific for each of the known pathogenic viruses, such as hepatitis A virus, hepatitis B virus, cytomegalovirus, EB virus, varicella virus and herpes simplex virus, and diagnosing the patient whose serum is negative with respect to the antibody specific for any of the above-mentioned viruses, as having NANB hepatitis, or by performing a histopathological examination through a biopsy of the liver ("Disease of the Liver and biliary system", 8th edition, S. Shenlock, pp. 326-333, 1989, Blackwell Scientific Publications). At the same time, another diagnosis method has also been used. For example, there have been used a method in which the activity of an enzyme in serum, such as GPT [glutamic-pyruvic transaminase, also known as "ALT" (alanine aminotransaminase)], GOT [glutamic-oxalo-acetic transaminase, also known as "AST" (aspartate aminotransferase)], and guanine deaminase (also known as "guanase") is determined ("Kan Tan Sui (Liver, Gallbladder, Pancreas)", Vol. 14, pp. 519-522, 1987). With respect to the GPT or GOT in serum mentioned above, a standard for the diagnosis of NANB hepatitis in which lasting and abnormally high activities of GPT and GOT are utilized as a criterion for the diagnosis of NANB hepatitis, is employed in Japan ("Journal of Blood Transfusion Society in Japan", Vol. 31, No. 4, pp. 316-320, 1985; and "Nippon Rinsho", Vol. 46, p. 2635-2638, 1988). Regarding the immunological diagnosis, in the present situation in which the isolation and identification of NANBV are difficult, an antigen-antibody reaction between an antigen obtained by expression of NANBV cDNA clone (which has been isolated using the techniques of genetic engineering and the knowledge of immunology) and the serum of an NANB hepatitis patient is used as a criterion. Examples of known antigens include an expression product of an NANBV cDNA prepared from the plasma of an NANB hepatitis patient (European Patent Application Publication No. 363025), an expression product of "HCV" cDNA prepared from the plasma of a chimpanzee having the symptoms of NANB hepatitis (European Patent Application Publication No. 318216 and Japanese Patent Application Laid-Open Specification No. 2-500880), an expression product of an NANBV cDNA derived from the liver of an NANBV-infected chimpanzee (European Patent Application Publication No. 293274, Japanese Patent Publication Specification No. 64-2576 and Japanese Patent Application Laid-Open Specification No. 1-124387). As a method for determining the antigen-antibody reaction, RIA (radioimmunoassay) and EIA (enzyme immunoassay) are generally used. However, these expression products are different in antigenicity. The antigen which is an expression product of HCV cDNA (that is, the C-100 antigen mentioned above) can be some criterion or yardstick for the diagnosis of chronic hepatitis caused by the HCV infection. However, since the region in which the antigen (C-100) exhibits its antigenicity is limited ("Biseibutsu (Microorganism)", Vol. 5, pp. 463-475, 1989; "Kan Tan Sui (Liver, Gallbladder, Pancreas)", Vol. 20, pp. 47-51, 1990; and "Igaku-no Ayumi (Progress of Medicine)", Vol. 151, p. 871, 1989), this antigen is unsatisfactory from the viewpoint of accurate diagnosis of NANB hepatitis and NANBV infection and from the viewpoint of accurate determination of the progress of a patient suffering from chronic hepatitis and acute hepatitis for treatment thereof. Therefore, it has been desired to obtain a reliable method for the diagnosis and prognosis of the NANB hepatitis.
(5) Therapy and Prevention: Recently, the usefulness of α- and β-interferons in the treatment of chronic NANB hepatitis have been reported ("Kan Tan Sui (Liver, Gallbladder, Panceras)" vol.20, pp. 59-64, 1990; "Igaku-no Ayumi (Progress of Medicine)", vol. 151, pp. 871-876, 1989). However, a suitable dose of α- and β-interferons and a suitable period for administration thereof have not yet been established.

On the other hand, for prevention of NANB hepatitis, various vaccines are used in which the above-mentioned conventional expression products of NANBV cDNAs (European Patent Application Publication No. 363025) or HCV cDNAs (European Patent Application Publication No. 318216) are used as an antigen. However, as is apparent from the fact that the NANBV itself has not yet been isolated and identified before completion of the present invention, it has been impossible to specify an antigen useful for NANBV vaccines from the above-mentioned expression products each having a variety of antigenic determinants (epitopes) and determine the effectiveness and safety of such a specific antigen so that the antigen can be clinically used. Accordingly, there is no NANBV vaccine which can be advantageously put into practical use.

### Summary Of The Invention

The present inventors have made extensive and intensive studies with a view toward solving the above-mentioned problems by developing a novel NANBV genomic cDNA. As a result, the present inventors have surprisingly succeeded in cloning an NANBV genomic cDNA, which not only has excellent reliability as compared to the known NANBV cDNA but also is larger in length than any known NANBV cDNAs and contains the entire region of the open reading frame of the NANBV genome, and expressing this NANBV cDNA to thereby obtain an NANBV antigen peptide which can reliably exhibit an antigen-antibody reaction specific for not only sera from patients having chronic NANB hepatitis but also sera from patients having acute NANB hepatitis. This success is attributed to a unique technique of the present inventors such that in order to obtain an authentic NANBV genome, NANBV RNAs are extracted directly from NANBV particles contained in whole blood of a patient having NANB hepatitis or a resected liver of a patient having NANB hepatitis and liver cancer in combination, without multiplying the NANBV in a chimpanzee having unknown factors which are considered to have rendered difficult the isolation of NANBV, although the amount of NANBV in the blood or resected liver is extremely small, that is, as small as about 1/10,000 that of a hepatitis A virus or a hepatitis B virus, but with paying minute care in the operating procedure so that the NANBV and its genome do not undergo cleavage and/or decomposition by the action of body fluids or blood enzymes during the storage of fresh materials for NANBV genome. RNAs thus prepared from fresh human materials are then converted to double-stranded cDNA by means of a reverse transcriptase to obtain a cDNA library. In order to screen an NANBV genome from the cDNA library, the cDNAs are individually inserted in lambda gt11 phage vectors and then expressed on the phage plaques at high concentration, followed by screening of NANBV genomic cDNAs by repeatedly conducting enzyme immunoassay (EIA) in which both serum from a convalescent patient having acute NANB nepatitis and serum from a patient having chronic NANB hepatitis are used. Thus, safe production of the NANBV antigen polypeptide with high purity on a large scale at low cost without biohazard, has for the first time been realized by expressing the cDNA of the present invention by recombinant DNA techniques. Based on the above, the present invention has been completed.

### Brief Description of the Drawings

In the Drawings:
Fig. 1(1) and Fig. 1(2) are diagrams showing the relationships between the cDNA clones of the NANBV gene of the present invention, shown relative to the entire region of the NANBV genome;
Fig. 2(1) through Fig. 2(16) show the nucleotide sequence of the entire region of the NANBV genomic cDNA according to the present invention and the amino acid sequence coded for by the nucleotide sequence; and
Fig. 3 is a diagram showing the hydrophobicity profiles of both of the NANBV of the present invention and the Japanese encephalitis virus (JEV), in which the hydrophobicity index of the NANBV is compared with that of the JEV.

### Detailed Description of the Invention

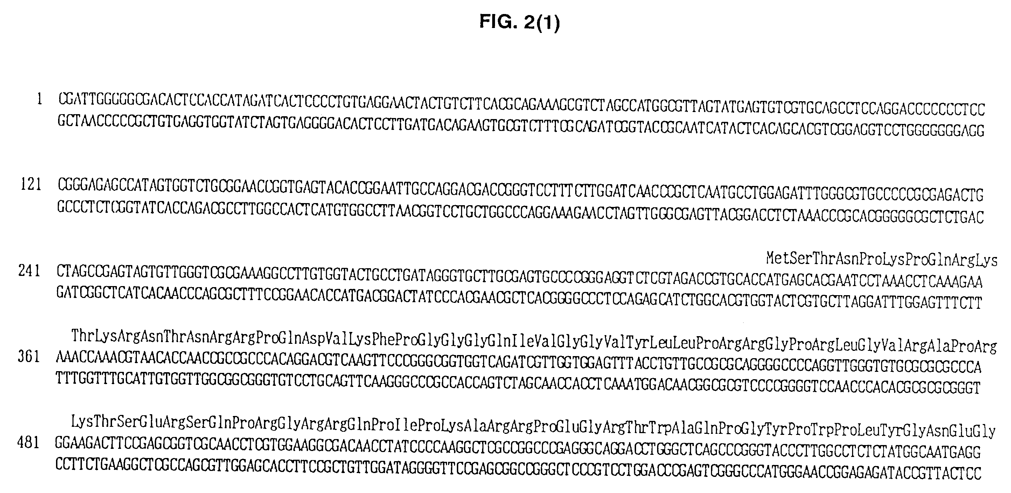

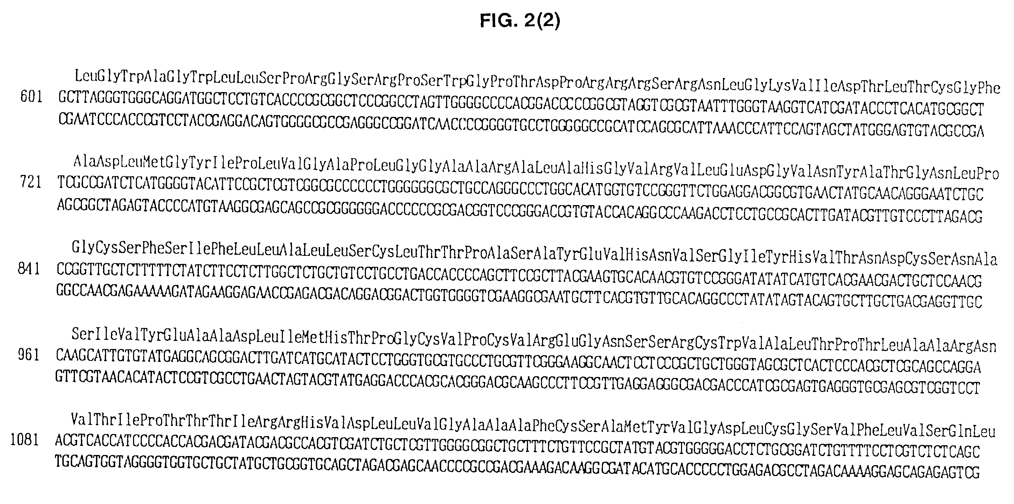

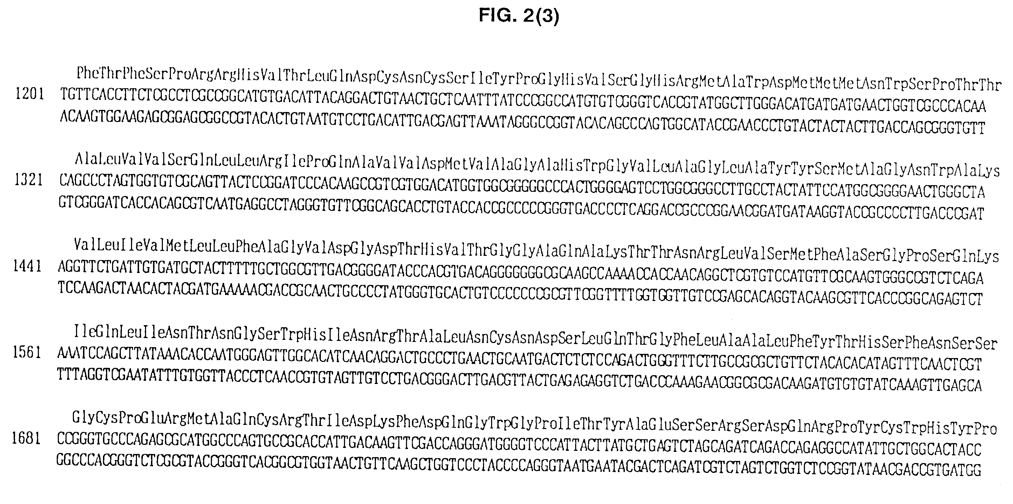

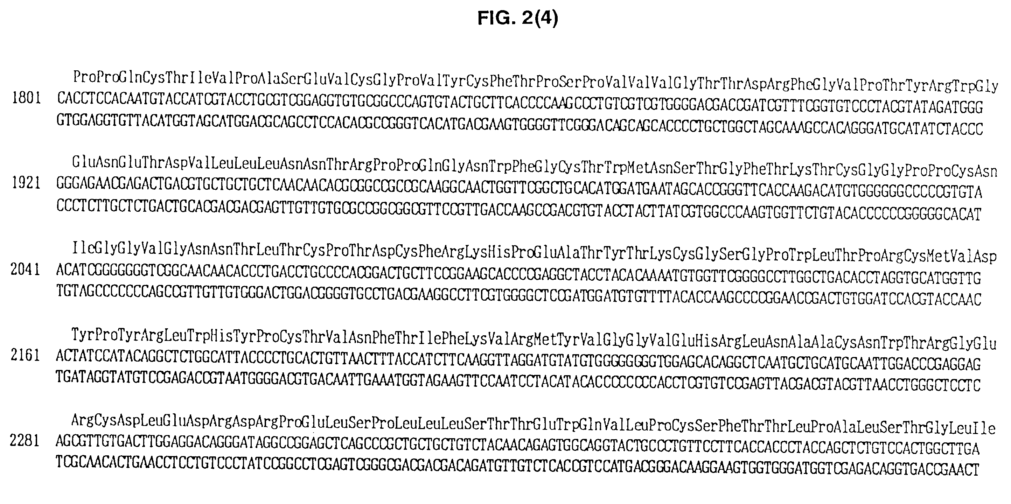

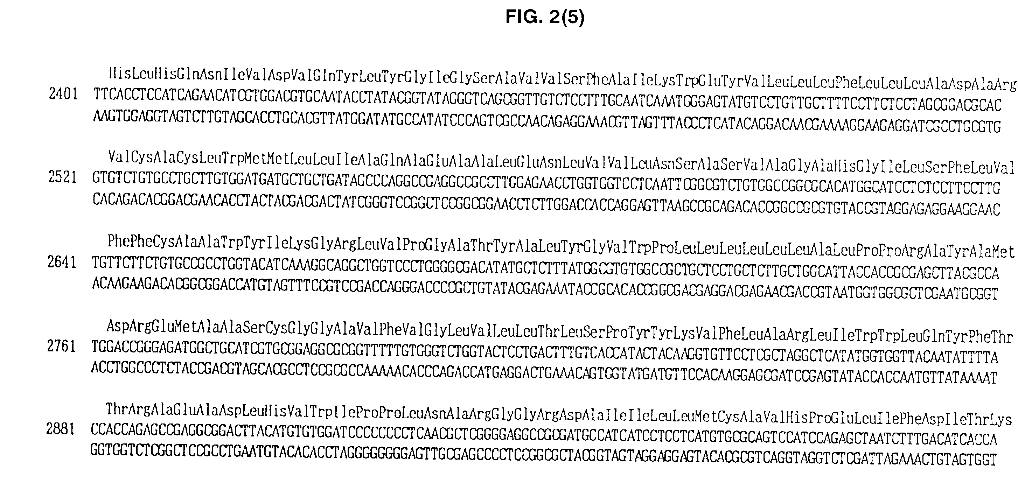

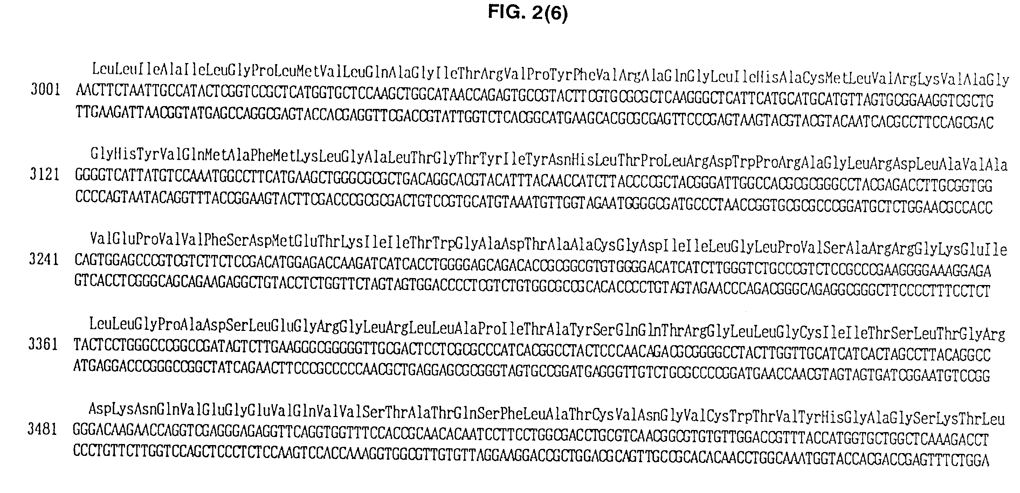

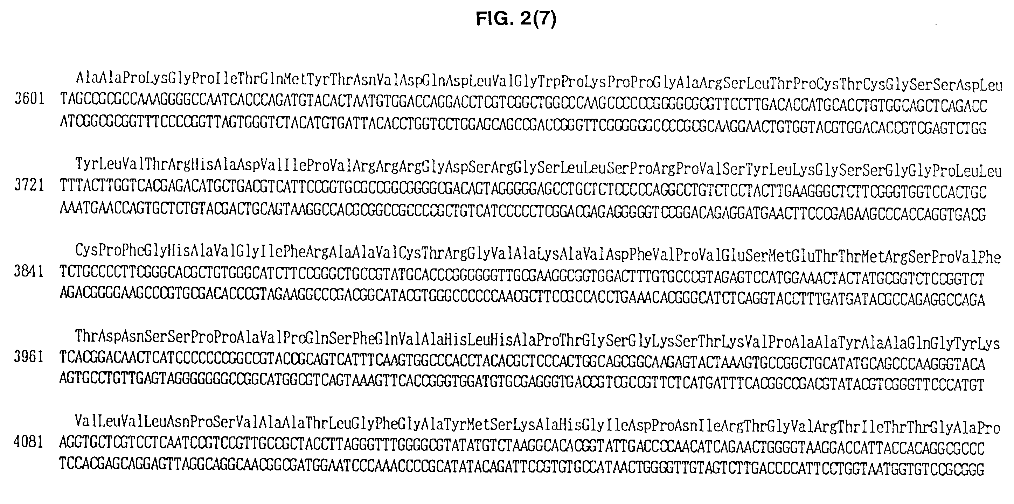

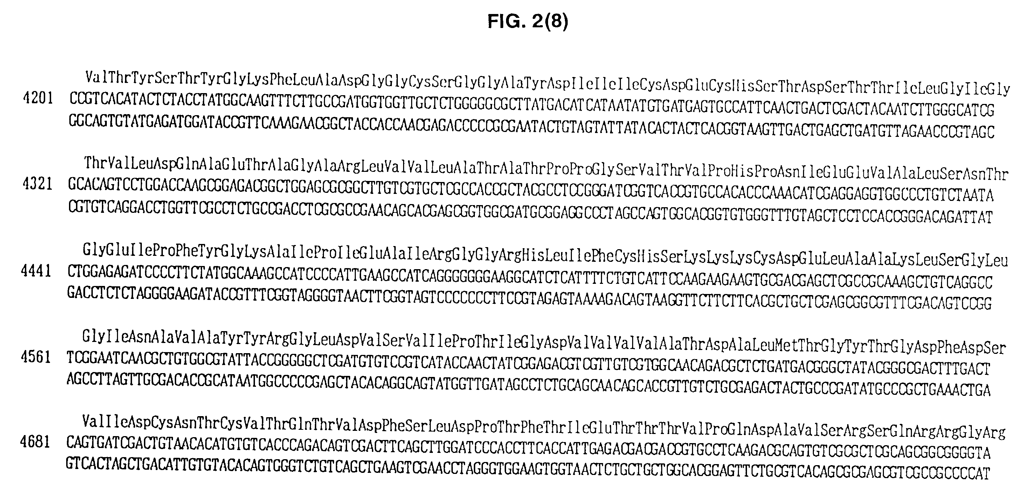

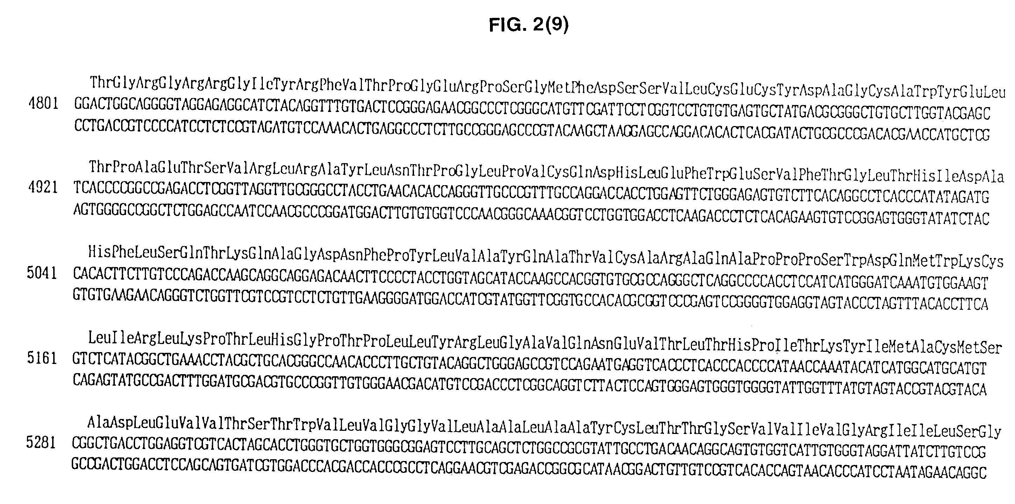

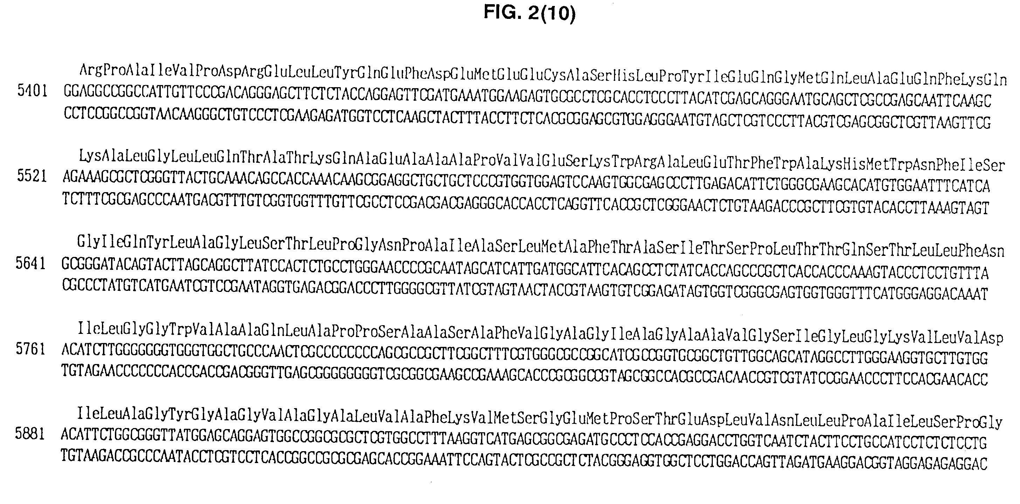

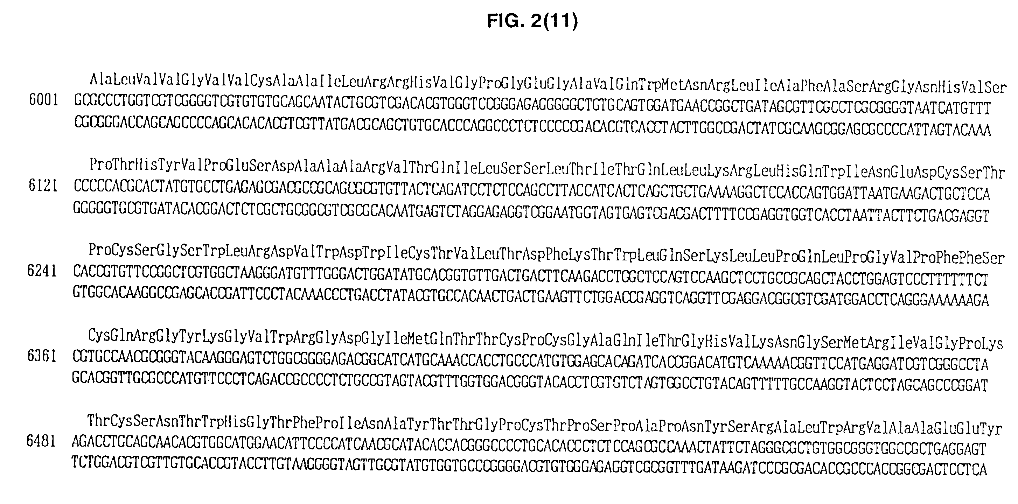

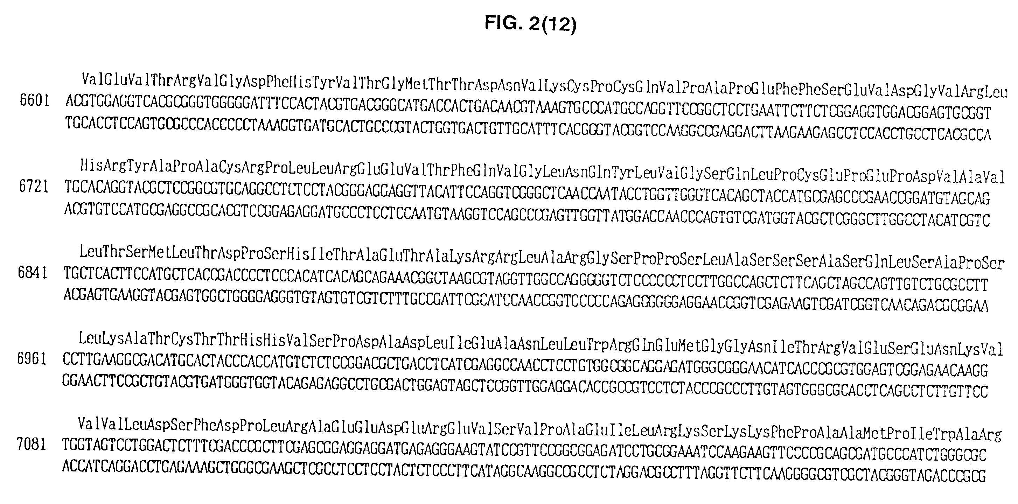

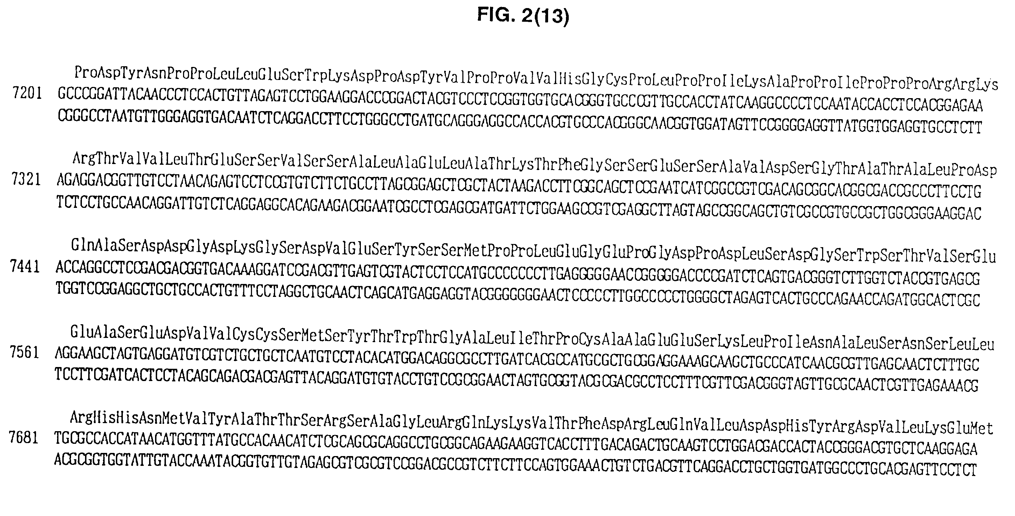

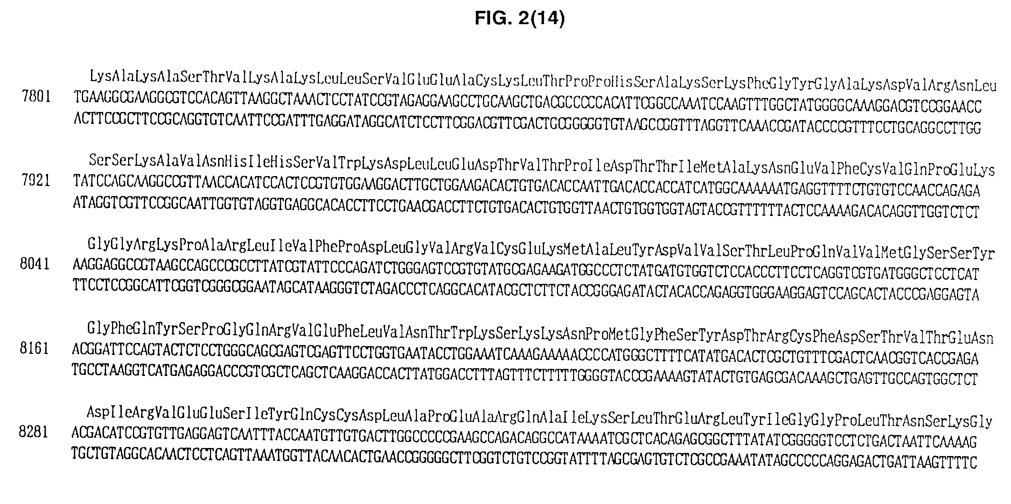

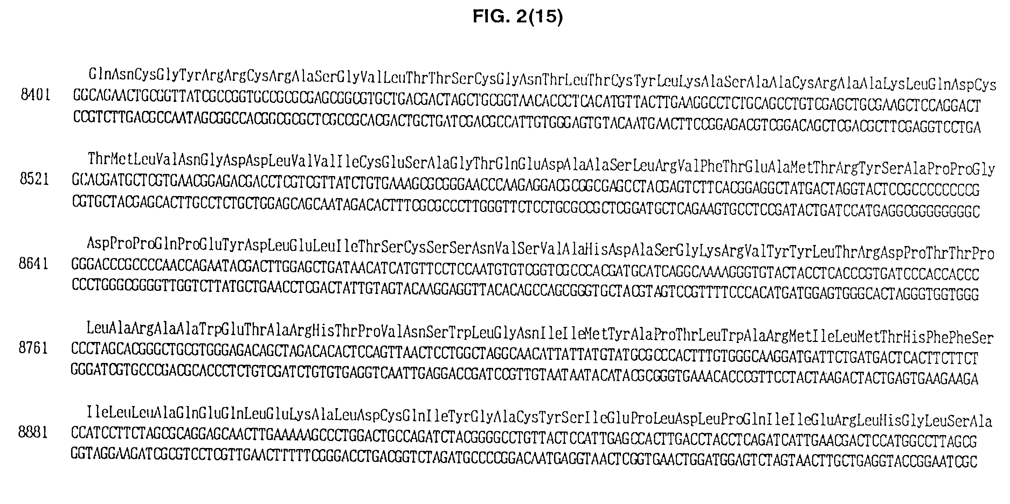

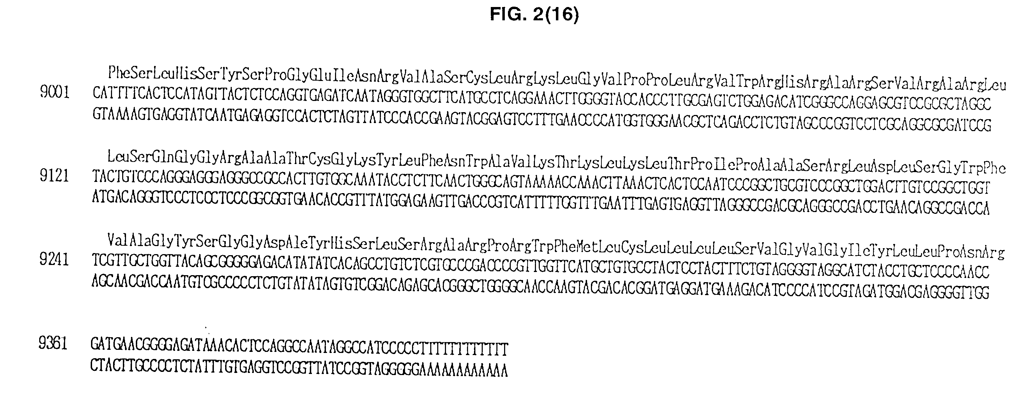

In its broadest aspect, the present invention provides an isolated DNA
(a) consisting of the following nucleotide sequence and a complementary sequence to said nucleotide sequence:
   a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
   or consisting of a nucleotide sequence obtained by substituting at least one nucleotide of said nucleotide sequence in accordance with the degeneracy of the genetic code; and
(b) comprising at least one nucleotide sequence selected from the following nucleotide sequences and respective complementary sequences to said nucleotide sequences:
   a nucleotide sequence of the 333rd to 677th nucleotides,
   a nucleotide sequence of the 333rd to 1499th nucleotides,
   a nucleotide sequence of the 333rd to 6371st nucleotides,
   a nucleotide sequence of the 333rd to 9362nd nucleotides,
   a nucleotide sequence of the 474th to 563rd nucleotides,
   a nucleotide sequence of the 678th to 905th nucleotides,
   a nucleotide sequence of the 906th to 953rd nucleotides,
   a nucleotide sequence of the 906th to 1499th nucleotides,
   a nucleotide sequence of the 1020th to 1046th nucleotides,
   a nucleotide sequence of the 1020th to 1121st nucleotides,
   a nucleotide sequence of the 1194th to 1232nd nucleotides,
   a nucleotide sequence of the 1209th to 1322nd nucleotides,
   a nucleotide sequence of the 1500th to 2519th nucleotides,
   a nucleotide sequence of the 2520th to 3350th nucleotides,
   a nucleotide sequence of the 3351st to 5177th nucleotides,
   a nucleotide sequence of the 4485th to 4574th nucleotides,
   a nucleotide sequence of the 5178th to 5918th nucleotides,
   a nucleotide sequence of the 5544th to 5633rd nucleotides,
   a nucleotide sequence of the 5919th to 6371st nucleotides,
   a nucleotide sequence of the 6372nd to 9362nd nucleotides.
Of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or comprising a nucleotide sequence obtained by substituting at least one nucleotide of said nucleotide sequence in accordance with the degeneracy of the genetic code.

In the present invention, unless otherwise specified, the left end and right end of the sequence of deoxyribonucleotides are the 5' end and 3' end, respectively. Further, unless otherwise specified, the left end and right end of the amino acid sequences of peptides are the N-terminus and C-terminus, respectively.

The DNA of the present invention and the polypeptide expression products thereof can be prepared and identified in accordance with the following steps (I) to (VIII).

Step (I): Selection and collection of a material for extracting an NANBV RNA.

As a material for extracting the NANBV RNA, there may be used, for example, blood, lymph, ascites and hepatocyte of an NANBV carrier, or of a human or a chimpanzee suffering from NANB hepatitis, and hepatocyte of a patient suffering from NANB hepatitis and liver cancer or hepatoma in combination. Since the materials derived from a chimpanzee may contain NANBV in a relatively small amount as compared to the materials derived from a human and a chimpanzee has unknown factors which are considered to have rendered difficult the isolation of NANBV, the use of the materials derived from a human is preferred. Of blood, lymph, ascites and hepatocytes from a human, blood can most easily be obtained in a large amount. For example, blood which is not acceptable for use as blood for transfusion is available from a blood bank in a large amount. Such blood can advantageously be used as a material for extracting an NANBV RNA. When blood is used as a material, blood is separated into plasma and erythrocytes. The thus obtained plasma is examined to determine whether or not the plasma is negative to the surface antigen of hepatitis B virus (WHO expert committee on viral hepatitis: Advances in viral hepatitis, WHO Technical Report Series, 602, 28-33, 1977) and negative to a genomic DNA of hepatitis B virus (Brechot, C., Hadchouel, M., Scotto, J., Degos, F., Charnay, P., Trepo, C., Tiollais, P.: Detection of hepatitis B virus DNA in liver and serum: a direct appraisal of the chronic carrier state. Lancet 2: 765-768, 1981). Further, the plasma is examined with respect to the activities of enzymes, such as GPT (Wroblewski, F. & LaDue, J. S.: Serum glutamic-pyruvic transaminase in cardiac and hepatic disease, Proc. Soc. Exp. Biol. Med., 91, 569, 1956), GOT, guanase and the like, which are employed as the criterion for the diagnosis of NANB hepatitis. The above-mentioned procedures of the separation of blood into plasma and erythrocytes and the examination of the plasma are conducted with respect to blood of different lots. The plasma which is negative to both surface antigen and genomic cDNA of hepatitis B virus and exhibits extremely high activities of the above-mentioned enzymes, for example, a GPT activity of 35 IU/litre or more, is pooled.

The number of the NANB hepatitis virus particles in blood is extremely small as compared to that of the hepatitis B virus particles as mentioned hereinbefore. From the results of the infection experiment, the number of the NANB hepatitis virus particles in blood is estimated to be about 1/10,000 of the number of the hepatitis B virus particles (Bradley, D.W., (1985): Research perspectives in post-transfusion non-A, non-B hepatitis, in "Infection, Immunity and Blood Transfusion", edited by Dodd, R.Y. & Barker, L.F., published by Alan R. Liss, Inc., New York, pp. 81-97). Therefore, for the extraction of the RNA, it is preferred to use blood in a large amount, for example, in an amount as large as about 3 to 10 liters. Fresh whole blood to be used as a material for extracting an NANB RNA from NANBV particles is stored at 1 to 5 °C in order to prevent NANBV and its gene from being denatured and to prevent its gene from being cleaved or decomposed by the action of an enzyme. It is also desirable to complete the preparation of NANBV RNAs by Step (II) within 48 to 72 hours from the collection of the fresh whole blood. When a hepatocyte is used as a material, about 1 to 3 g of a non-cancerous or a cancerous portion of a liver tissue resected from a patient having hepatoma or liver cancer which is a complication of a chronic NANB hepatitis may advantageously be used. Hepatocyte to be used as a material is stored in a frozen state at -70 °C.

### Step (II): Preparation of the NANBV RNA

From the material obtained in Step (I), the RNA may be extracted and purified by conventional methods. For example, when fresh whole blood is used as the material, about 2 to 10 liters of fresh whole blood is subjected to low-speed centrifugation to collect a plasma fraction as a supernatant. The virus fraction is obtained from the plasma through purification for use in the subsequent procedure for the extraction and purification of the RNA.

On the other hand, when hepatocyte is used as a material for extracting the NANBV RNA, about 5 to 30-fold volume of a diluent containing ribonuclease inhibitor is added to the liver tissue. Then, according to the conventional method using a homogenizer and the like, the liver tissue is crushed or disrupted to obtain a homogenate of hepatocyte. As a diluent, 10 to 150 mM of a conventional buffer may be used. Then, the homogenate is subjected to low-speed centrifugation to collect a supernatant. The collected supernatant is used as an original solution for the extraction and purification of the NANBV RNA. The extraction and purification of the NANBV RNA may be conducted by the conventional method, for example, an extraction method in which a mixture of a ribonuclease inhibitor, such as heparin, diethyl pyrocarbonate and guanidine thiocyanate, with a surfactant, a chelating agent, or a reducing agent capable of enhancing the denaturation of a protein, is used; a method in which fractionation is conducted by density gradient centrifugation using sucrose, cesium chloride, cesium trichloroacetate, Ficoll (Pharmacia Fine Chemicals AB, Sweden) or the like as a solute of a gradient; a method in which separation is conducted by affinity column utilizing the 3'-terminal poly A chain which an mRNA specifically has; a separation method in which an mRNA-bonded polysome is obtained by the immunoprecipitation using an antibody specific for a protein synthesized on the polysome; a phenol extraction method based on a principle of two-phase separation; a precipitation method by the use of a polyethylene glycol, a dextran sulfate, an alcohol or the like. The above-mentioned methods may be used individually or in combination. The above-mentioned procedure for extracting and purifying the NANBV RNA may preferably be conducted at pH 3 to 10 in order to prevent the irreversible denaturation of the RNA.

### Step (III): Preparation of a double-stranded cDNA from the NANBV RNA

Using the above-obtained NANBV RNA as a template, a cDNA may be prepared by a customary method. That is, using an oligodeoxythymidine and a random hexanucleotide primer as primers and using a reverse transcriptase, a cDNA complementary to the NANBV RNA is synthesized using the NANBV RNA as a template to obtain a double-strand comprising the cDNA and the NANBV RNA which are complementarily bonded to each other. Then, the thus obtained double-strand is reacted with ribonuclease H so that the NANBV RNA is decomposed and removed from the cDNA. Thus, a single-stranded cDNA is obtained. Using the obtained single-stranded cDNA as a template, a double-stranded cDNA is synthesized by means of a DNA synthase. The double-stranded cDNA synthesis may easily be conducted using a commercially available kit for cDNA synthesis, for example, cDNA Synthesis System Plus® (manufactured and sold by Amersham, England), cDNA System Kit® (manufactured and sold by Pharmacia LKB, Sweden), cDNA Synthesis Kit® (manufactured and sold by Boehringer Mannheim GmbH, West Germany), and the like. When the quantity of the synthesized cDNA is small, the cDNA can be amplified using a conventional method, such as PCR (polymerase chain reaction) method ("PCR Technology", edited by H.A. Erlich, published by Stockton Press, 1989) using a PCR kit, such as AmpliTaq (manufactured and sold by Perkin Elmer Cetus, U.S.A.).

### Step (IV): Preparation of a cDNA library

Using the cDNA prepared in Step (III), a cDNA library is prepared by a customary method. That is, the cDNA prepared in Step (III) is cut into fragments having different lengths and the resultant various cDNA fragments are individually ligated to replicable cloning vectors, to thereby obtain a cDNA library. As a replicable cloning vector, any known or commercially available vectors, such as phage genes, cosmids, plasmids and animal virus genes may be used. When a phage gene or a cosmid is used as a replicable vector, in order to attain high stability and high transforming ability of the vector after each of the cDNA fragments has been individually inserted therein, the in vitro packaging of each of the cDNA-inserted vectors is conducted by a customary method. Thus, the cDNA-inserted vectors are obtained in the form of a recombinant phage particle. The obtained phage particles are used as a cDNA library for cDNA cloning. On the other hand, when a plasmid is used as a replicable vector, the above-mentioned cDNA fragments are individually inserted in the plasmid vectors and the resultant cDNA-inserted vectors are then individually introduced into host cells, such as cells of Escherichia coli, Bacillus subtilis, yeast or the like, according to a customary method. The thus obtained transformants are used as a cDNA library for cDNA cloning. Further, when the animal virus gene is used as a replicable vector, the above-mentioned cDNA fragments are individually inserted in the virus gene vectors and the resultant recombinant viruses are then individually transfected into sensitive animal cells according to a standard method and multiplied in the cells. In the case of the recombinant virus, the obtained recombinant viruses as such are used as a cDNA library.

The preparation of the cDNA library may easily be conducted using a commercially available kit, for example, a cDNA cloning system lambda gt10 and lambda gt11 (manufactured and sold by Amersham, England; BRL Inc., U.S.A.; and Stratagene Inc., U.S.A.), an in vitro packaging system (manufactured and sold by Amersham, England; BRL Inc., U.S.A.; and Stratagene Inc., U.S.A.) and the like.

### Step (V): Cloning of a cDNA containing an NANBV gene from the cDNA library

In this step, a cDNA clone containing an NANBV gene is obtained. When the cDNA library is comprised of transformants, the transformants are cultured on a standard agar medium to form colonies. On the other hand, when the cDNA library is comprised of recombinant phage particles or recombinant viruses, these phage particles or recombinant viruses are used to infect known sensitive host cells, such as Escherichia coli, Bacillus subtilis, yeast, animal cell culture and the like, and cultured to form a plaque, or to multiply the infected cells. The above-obtained transformant colonies, plaques or infected cells are subjected to immunoassay by at least one of the standard methods individually using serum from a convalescent patient having acute NANB hepatitis, serum from a patient having chronic NANB hepatitis, and serum from chimpanzee infected with an NANBV irrespective of whether or not the NANBV is of the type which causes a tubular structure to be formed in the cytoplasm of the hepatocyte of the chimpanzee, so that colonies, plaques or infected cells which have produced an NANBV antigen specifically reacted with at least one of the above-mentioned sera are selected and isolated. For the strict selection of the colonies, plaques and infected cells, it is preferred that the above procedure be repeated. From each of the thus selected and isolated colonies, plaques or the infected cells, a cDNA clone containing an NANBV gene is isolated according to a standard method described in T. Maniatis et al., Molecular Cloning, A Laboratory Manual, published by Cold Spring Harbor Laboratory, U.S.A., pp. 309-433 (1982). The immunoassay may be conducted by, for example, an enzyme-labeled antibody technique in which an antibody labeled with an enzyme, such as peroxidase and alkaline phosphatase is used; and a fluorescent antibody technique in which an antibody labeled with fluorescein isothiocyanate, europium or the like is used. It is preferred that the immunoassay by the above-mentioned technique be conducted by an indirect method because with the indirect method, high sensitivity immunoassay can be attained even by the use of an extremely small amount of serum from a patient. As a primary antibody to be used in the indirect method, serum from a patient having NANB hepatitis or serum from a chimpanzee having NANB hepatitis may preferably be employed because these sera contain an antibody specific for an NANBV antigen in relatively large amount. As a secondary antibody to be used in the indirect method, a commercially available anti-human Ig (immunoglobulin) antibody labeled with an enzyme, a fluorescent substance or the like may be used.

A specimen to be subjected to immunoassay may be prepared according to a conventional method, for example, a blotting method in which nucleic acids and proteins of the colonies, plaques and infected cells are adsorbed on a filter membrane, a method in which a microplate or a slide glass for microscopy is used, or the like. When the blotting method is used in combination with an indirect, enzyme-labeled antibody technique, the selection of the intended colonies, plaques or infected cells from an extremely large number of the original colonies, original plaques or original infected cells can be conducted easily and promptly. In this case, blotting is conducted by contacting a commercially available filter made of nitrocellulose, cellulose acetate, nylon or the like, with the colonies, plaques or infected cells.

The above-obtained cDNA clone is a part of the NANBV gene. Therefore, in order to obtain cDNA clones covering the entire region of the NANBV gene, it is requisite to extend the cNDA clone by a method in which cDNA fragments adjacent to the cDNA clone are isolated by using 3'- and 5'- terminals of the cDNA clone as a probe. In this case, the technique which is known as "gene walking" (also known as "genomic walking" or "chromosome walking") may be employed ("DNA cloning volume III", edited by D.M. Glover, pp.37-39, IRL Press, 1987; "Molecular Cloning - a laboratory manual" 2nd edit., T. Maniatis et al, 3.21 - 3.23, 1989). By the repetition of the cloning procedure and the gene walking, the entire region of the NANBV gene can be obtained in the form of cDNA clones.

In this step, it is preferred to determine the nucleotide sequence of each of the obtained cDNA clones. The determination of the nucleotide sequence of the cDNA clone may generally be conducted according to a conventional method, for example, the Maxam-Gilbert method, the dideoxy chain termination method (Analytical Biochemistry, 152, 232-238, 1986), or the like.

Based on the determined nucleotide sequence, the amino acid sequence can be determined. The sequencing of the amino acids is conducted from the location of the initiation codon (ATG on the cDNA or AUG on the mRNA). Important portions of the amino acid sequence, for example, a hydrophilic portion, which is considered to constitute an epitope, can be identified by synthesizing a peptide corresponding to each hydrophilic portion and purifying the synthesized polypeptide by high performance liquid chromatography (HPLC), followed by subjecting the purified peptide to enzyme immunoasssy (EIA) or radioimmunoassay (RIA).

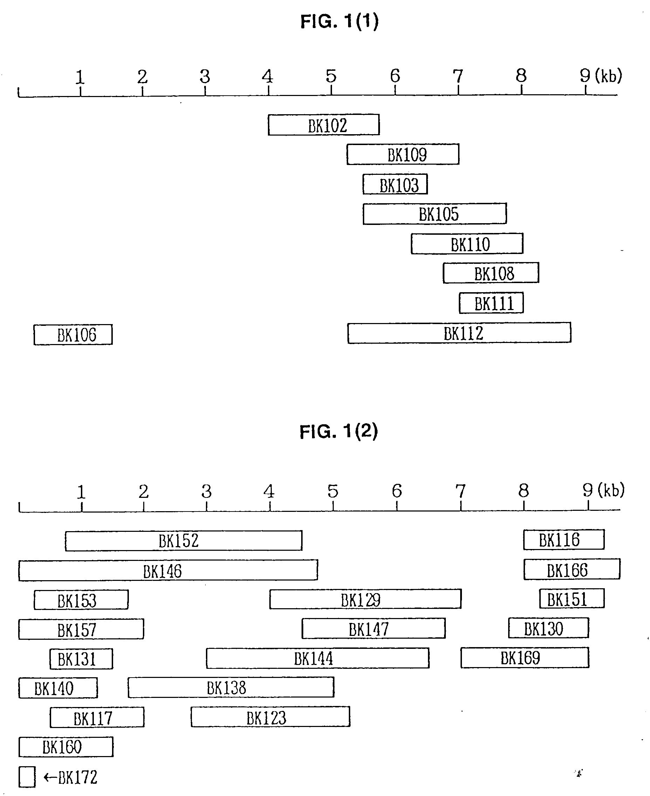

The cDNA clones are preferably classified into groups according to the respective properties of the NANBV antigen polypeptides coded for by the cDNA clones in order to distinguishing clones from one another. In this connection, the location of each cDNA clone on the restriction map of the NANBV gene can be used as a yardstick for the classification [see Fig. 1(1) and Fig. 1(2)]. Further, it has been found that some of NANBVs have the ability to cause a tubular structure to be formed in the cytoplasm of a hepatocyte of a chimpanzee, and some of NANBV do not have such ability (Science, 205, pp. 197-200, 1979). Therefore, the cDNA clones may be identified and classified by examining the serological reactivity of each cDNA clone with serum from a chimpanzee infected with an NANBV of the type which causes a tubular structure to be formed in the cytoplasm of the hepatocyte of the chimpanzee and with serum from a chimpanzee infected with an NANBV of the type which does not cause a tubular structure to be formed in the cytoplasm of the hepacyte of the chimpanzee. The examination of this serological reactivity may be conducted by immunoassay mentioned above.

In the present invention, as shown in Figs. 1(1) and 1(2), the cDNA clones of the NANBV gene of the present invention are identified with prefix "BK".

Fig. 1(1) is a diagram showing the relationships between the cDNA clones of the NANBV gene of the present invention, shown relative to the entire region of the NANBV gene, and Fig. 1(2) is a diagram showing the relationships between the cDNA clones obtained by gene walking, shown relative to the entire region of the NANBV gene.

These BK NANBV cDNA clones include, for example, Escherichia coli BK 108 (deposited at Fermentation Research Institute, Japan under the accession number FERM BP-2971), Escherichia coli BK 129 (deposited at Fermentation Research Institute, Japan under the accession number FERM BP-2972), Escherichia coli BK 138 (deposited at Fermentation Research Institute, Japan under the accession number FERM BP-2973), Escherichia coli BK 153 (deposited at Fermentation Research Institute, Japan under the accession number FERM BP-2974), Escherichia coli BK 157, Escherichia coli BK 166 (deposited at Fermentation Research Institute, Japan under the accession number FERM BP-2975), and Escherichia coli BK 172 (deposited at Fermentation Research Institute, Japan under accession number FERM BP-2976). These seven BK NANBV cDNA clones are considered to cover at least the entire region of the open reading frame of the NANBV gene and probably the entire region of the NANBV gene.

The nucleotide sequence of the entire region of the NANBV gene which is covered by the above-mentioned BK NANBV cDNA clones and the amino acid sequence which is coded for by this nucleotide sequence are shown in Fig. 2(1) through Fig. 2(16). Based on the entire NANBV nucleotide sequence and the entire NANBV amino acid sequence shown in Fig. 2(1) through Fig. 2(16), various studies and observations can be made with respect to the homology of the nucleotide sequence and amino acid sequence of the NANBV gene to those of other virus genes, the hydrophobicity index (hydrophobicity/hydrophilicity profile), the structure of the NANBV gene, the regions of epitopes (antigenic determinants) and the like.

With respect to the homology, studies can be made by comparison of the nucleotide sequence and amino acid sequence of the NANBV gene with those of various viruses whose genes are well known (Japanese Patent Application Laid-Open specification No. 62-286930 and "Virology", Vol. 161, pp. 497-510, 1987) and those of other viruses, such as bovine virus diarrhea-mucosal disease virus ("Virology", Vol. 165, pp. 497-510, 1988), swine cholera virus ("Virology", Vol. 171, pp. 555-567, 1989), tobacco vein mottling virus ("Nucleic Acid Research, Vol. 165, pp. 5417-5430, 1986), etc.

With respect to the analysis of the hydrophobicity index, studies can be made by techniques using, for example, a genetic information processing software, SDC-Genetyx (manufactured and sold by SDC Software Co., Ltd., Japan), Doolittle's program (Journal of Molecular Biology, Vol. 157, pp. 105-132, 1982) and the like.

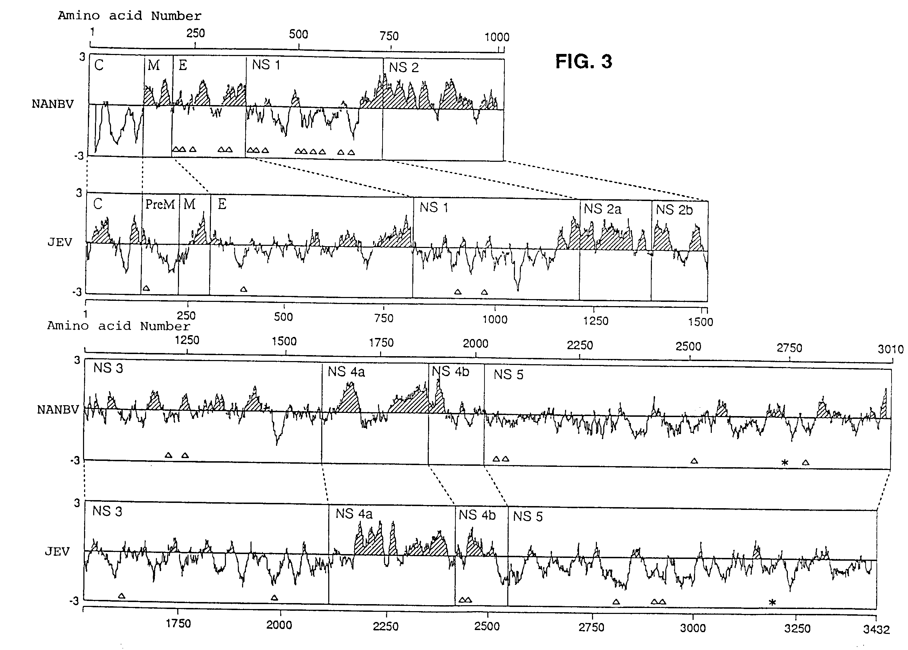

Fig. 3 is a diagram showing the hydrophobic profiles of both of the NANBV of the present invention and the Japanese encephalitis virus (JEV), in which the respective hydrophobic indexes of both viruses are compared with each other. In Fig. 3, the abscissa indicates the amino acid number, the ordinate indicates the hydrophobicity index, the vacant triangle indicates the glycosylation site, the asterisk indicates the site of amino acid sequence (Gly-Asp-Asp) common to RNA polymerase, and C, PreM, M, E and NS represent core protein, pre-matrix protein, matrix protein, envelope protein and non-structural protein, respectively. A significant similarity is found between the gene structure of the NANBV gene and that of the JEV gene. As shown in Fig. 3, the polypeptide of the NANBV of the present invention contains three structural proteins, namely, core protein (C), pre-matrix protein (PreM) that is further processed to matrix protein (M) and envelope protein (E), and seven nonstructural proteins, NS1, NS2a, NS2b, NS3, NS4a, NS4b and NS5. These proteins are, respectively, coded for by the following nucleotide sequences.
- C protein:: from the 333rd to 677th nucleotides
- M protein:: from the 678th to 905th nucleotides
- E protein:: from the 906th to 1499th nucleotides
- NS1 protein:: from the 1500th to 2519th nucleotides
- NS2 protein:: from the 2520th to 3350th nucleotides
- NS3 protein:: from the 3351st to 5177th nucleotides
- NS4a protein:: from the 5178th to 5918th nucleotides
- NS4b protein:: from the 5919th to 6371th nucleotides
- NS5 protein:: from the 6372nd to 9362nd nucleotides

These nucleotide sequences are useful for the diagnosis of NANB hepatitis. Polypeptides respectively coded for by these nucleotide sequences are useful as antigens for not only vaccines but also diagnostic reagents for NANB hepatitis.

The above-mentioned three structural proteins are represented by the 1st(Met) to 389th(Gly) amino acids shown in Fig. 2(1) through Fig. 2(3). The 1st methionine residue is the residue that is coded for by the initiation codon.

By further studies by the present inventors, it has been found that the following nucleotide sequences contain epitopes which are reactive to an anti-NANBV antiboby:
nucleotide sequences respectively of the 333rd to 422nd nucleotides, of the 333rd to 1499th nucleotides, of the 333rd to 6371st nucleotides, of the 474th to 563rd nucleotides, of the 906th to 953rd nucleotides, of the 1020th to 1046th nucleotides, of the 1020th to 1121st nucleotides, of the 1194th to 1232nd nucleotides, of the 1209th to 1322nd nucleotides, of the 4485th to 4574th nucleotides and of the 5544th to 5633rd nucleotides.

As described hereinbelow, the above-mentioned nucleotide sequences or nucleotide sequences containing such nucleotide sequences as part of the whole sequences, can be effectively used not only for producing NANBV antigen polypeptides by recombinant DNA technique or chemical synthesis but also for diagnosing NANB hepatitis by hybridization or polymerase chain reaction (PCR).

Further, it has been found that a first nucleotide sequence comprising at least six nucleotides of the entire region from the 1st to 9416th nucleotides shown in Fig. 2(1) through Fig. 2(16) is useful as a probe for hybridization or as a primer for polymerase chain reaction in the diagnosis of NANB hepatitis and that a polypeptide comprising at least four amino acids, which is coded for by a nucleotide sequence of at least twelve nucleotides of the nucleotide sequence of the 333rd to 9362nd nucleotides is effective as an antigen not only for a vaccine but also for a diagnostic reagent for NANB hepatitis. Further, as is well known in the art, a second nucleotide sequence complementary to the first nucleotide sequence is also useful as a probe for hybridization or as a primer for polymerase chain reaction in the diagnosis of NANB hepatitis. Further, a nucleotide sequence obtained by substituting at least one nucleotide of at least part of the coding region of the first nucleotide sequence of the NANBV in accordance with the degeneracy of the genetic code can also be used for producing the antigen polypeptide of the present invention by recombinant DNA technique.

Accordingly, the isolated deoxyribonucleic acid of the present invention comprises at least one nucleotide sequence selected from the group consisting of a first nucleotide sequence comprising at least part of the non-A, non-B hepatitis virus entire nucleotide sequence from the 1st to 9416th nucleotides shown in Fig. 2(1) through Fig. 2(16) hereof and a second nucleotide sequence complementary to the first nucleotide sequence, or comprises at least one nucleotide sequence obtained by substituting at least one nucleotide of the first nucleotide sequence in accordance with the degeneracy of the genetic code.

In one preferred embodiment of the present invention with respect to the deoxyribonucleic acid, the first nucleotide sequence comprises at least six nucleotides of the non-A, non-B hepatitis virus entire nucleotide sequence from the 1st to 9416th nucleotides shown in Fig. 2(1) through Fig. 2(16) hereof.

In another preferred embodiment of the present invention with respect to the deoxyribonucleic acid, the first nucleotide sequence comprises at least one nucleotide sequence selected from the group consisting of a nucleotide sequence of the 333rd to 422nd nucleotides, a nucleotide sequence of the 333rd to 677th nucleotides a nucleotide sequence of the 333rd to 1499th nucleotides, a nucleotide sequence of the 333rd to 6371st nucleotides, a nucleotide sequence of the 474th to 563rd nucleotides, a nucleotide sequence of the 678th to 905th nucleotides, a nucleotide sequence of the 906th to 953rd nucleotides, a nucleotide sequence of the 906th to 1499th nucleotides, a nucleotide sequence of the 1020th to 1046th nucleotides, a nucleotide sequence of the 1020th to 1121st nucleotides, a nucleotide sequence of the 1194th to 1232nd nucleotides, a nucleotide sequence of the 1209th to 1322nd nucleotides, a nucleotide sequence of the 1500th to 2519th nucleotides, a nucleotide sequence of the 2520th to 3350th nucleotides, a nucleotide sequence of the 3351st to 5177th nucleotides, a nucleotide sequence of the 4485th to 4574th nucleotides, a nucleotide sequence of the 5178th to 5918th nucleotides, a nucleotide sequence of the 5544th to 5633rd nucleotides, a nucleotide sequence of the 5919th to 6371st nucleotides, a nucleotide sequence of the 6372nd to 9362nd nucleotides and a nucleotide sequence from the 1st to 9416th nucleotides.

The isolated antigen polypeptide of the present invention comprises at least one amino acid sequence comprising at least part of an amino acid sequence coded for by a deoxyribonucleic acid comprising a coding region from the 333rd to 9362nd nucleotides of the non-A, non-B hepatitis virus nucleotide sequence shown in Fig. 2(1) through Fig. 2(16) hereof.

In one preferred embodiment of the present invention with respect to the antigen polypeptide, the antigen polypeptide comprises at least one amino acid sequence of at least four amino acids, which is coded for by a nucleotide sequence of at least twelve nucleotides of the nucleotide sequence of the 333rd to 9362nd nucleotides.

In another preferred embodiment of the present invention with respect to the antigen polypeptide, the antigen polypeptide comprises an amino acid sequence coded for by a nucleotide sequence selected from the group consisting of a nucleotide sequence of the 333rd to 422nd nucleotides, a nucleotide sequence of the 333rd to 677th nucleotides, a nucleotide sequence of the 333rd to 1499th nucleotides, a nucleotide sequence of the 333rd to 6371st nucleotides, a nucleotide sequence of the 474th to 563rd nucleotides, a nucleotide sequence of the 678th to 905th nucleotides, a nucleotide sequence of the 906th to 953rd nucleotides, a nucleotide sequence of the 906th to 1499th nucleotides, a nucleotide sequence of the 1020th to 1046th nucleotides, a nucleotide sequence of the 1020th to 1121st nucleotides, a nucleotide sequence of the 1194th to 1232nd nucleotides, a nucleotide sequence of the 1209th to 1322nd nucleotides, a nucleotide sequence of the 1500th to 2519th nucleotides, a nucleotide sequence of the 2520th to 3350th nucleotides, a nucleotide sequence of the 3351st to 5177th nucleotides, a nucleotide sequence of the 4485th to 4574th nucleotides, a nucleotide sequence of the 5178th to 5918th nucleotides, a nucleotide sequence of the 5544th to 5633rd nucleotides, a nucleotide sequence of the 5919th to 6371st nucleotides, a nucleotide sequence of the 6372nd to 9362nd nucleotides and a nucleotide sequence of the 333rd to 9362nd nucleotides.

Furthermore, it should be noted that since a polypeptide coded for by the entire coding region of the NANBV shown in Fig. 2(1) through Fig. 2(16), or an expression product of the entire coding region contains all protein particles of the NANBV, such a polypeptide has a broad antigen-antibody reaction spectrum and therefore can react to a wide variety of antibodies produced by infection with NANB hepatitis virus as compared to an antigen containing a single epitope, so that it has high sensitivity in detecting NANB hepatitis.

### Step (VI): Expression of the NANBV genomic cDNA clone and a mass production of an NANBV antigen polypeptide.

In order to express the cloned cDNA of an NANBV antigen gene to produce an NANBV antigen polypeptide on a commercial scale, part or whole of the cloned cDNA present in the cDNA clone is taken out from the replicable cloning vector and recombined with a replicable expression vector. Illustratively stated, part or whole of the cDNA of each cDNA clone is cut off using a restriction enzyme to obtain a DNA fragment containing an NANBV antigen gene (hereafter referred to as "NANBV DNA fragment"). The NANBV DNA fragment is then inserted in a replicable expression vector by a customary method. When one DNA fragment is inserted in an expression vector, one type of antigen polypeptide can be produced by gene expression. When two or more of different DNA fragments are inserted in sequence in an expression vector, an antigen polypeptide can be produced by gene expression in the form of a fused polypeptide comprising polypeptides coded for by the inserted DNA fragments.

Further, a complete NANBV particle can be produced using an expression vector having inserted therein the entire region of the open reading frame (ORF) shown in Fig. 2(1) through Fig. 2(16). Furthermore, an incomplete NANBV particle containing no RNA genome can be produced using an expression vector having inserted therein the open reading frame which is deficient only in the NS5 region coding for RNA polymerase.

As the replicable expression vector which may be used in this step, any conventionally known or commercially available expression vector can be used. Examples of expression vectors include plasmid vector pSN508 for enterobacteria (U.S. Patent No. 4,703,005), plasmid vector pBH103 for yeast, and its series (Japanese Patent Application Laid-Open Specification No. 63-22098), plasmid pJM105 (Japanese Patent application Laid-Open Specification No. 62-286930), an attenuated chicken pox virus gene (Japanese Patent Application Laid-Open Specification No. 53-41202), an attenuated Marek's disease virus (The Journal of Japanese Society of Veterinary, 27, 20-24 (1984), and Gan Monograph on Cancer Research, 10, 91-107 (1971)), plasmid pTTQ series (manufactured and sold by Amersham, England), plasmid pSLV series (manufactured and sold by Pharmacia LKB, Sweden), and the like.

The NANBV DNA-inserted expression vectors are individually introduced or transfected into host cells sensitive to the vector according to a conventional method, to obtain transformants. Then, from the transformants, the transformant(s) which has produced an NANBV antigen polypeptide or an NANBV particle is selected. The production of an NANBV antigen polypeptide (or an NANBV particle) may be detected by the immunoassay mentioned above in Step (V). When an animal virus gene is used as an expression vector, a recombinant virus having an NANBV antigen polypeptide on the surface thereof may be obtained. Such a recombinant virus may advantageously be used as a raw material for a multifunctional vaccine having not only an antigenicity inherent in the virus vector but also an antigenicity of the NANBV.

By culturing the transformant or recombinant virus obtained above according to a customary method, an NANBV antigen polypeptide can be produced in the culture of the transformant or recombinant virus on a commercial scale. With respect to the details of the method in which an animal virus gene is used as an expression vector, reference may be made to European patent Application Publication No. 0 334 530 A1.

Accordingly, in still another aspect of the present invention, there is provided a method for producing a non-A, non-B hepatitis virus antigen polypeptide, which comprises:
(a) inserting a deoxyribonucleic acid into a replicable expression vector selected from a plasmid and an animal virus gene to obtain a replicable recombinant DNA comprising the plasmid and the deoxyribonucleic acid inserted therein when the replicable expression vector is a plasmid or obtain a recombinant virus comprising the animal virus gene and the deoxyribonucleic acid inserted therein when the expression vector is an animal virus gene,
   the deoxyribonucleic acid comprising a nucleotide sequence selected from the group consisting of a first nucleotide sequence comprising at least part of a region from the 1st to 1499th nucleotides or at least part of a region from the 1500th to 9416th nucleotides of the non-A, non-B hepatitis virus entire nucleotide sequence shown in Fig. 2(1) through Fig. 2(16) hereof and a nucleotide sequence obtained by substituting at least one nucleotide of the first nucleotide sequence in accordance with the degeneracy of the genetic code;
(b) transfecting cells of a microorganism or eukaryotic cell culture with the recombinant DNA when the replicable expression vector used in step (a) is a plasmid, to thereby form a transformant, followed by selection of the transformant from parent cells of the microorganism or eukaryotic cell culture;
(c) culturing the transformant obtained in step (b) to thereby express the deoxyribonucleic acid and produce a non-A, non-B hepatitis virus antigen peptide, or culturing the recombinant virus obtained in step (a) to thereby express the deoxyribonucleic acid and the animal virus gene and produce a non-A, non-B hepatitis virus antigen peptide in the form of a multiplied recombinant virus comprising an animal virus and a non-A, non-B hepatitis virus antigen peptide contained on the surface thereof; and
(d) isolating the non-A, non-B hepatitis virus antigen peptide alone or in the form of the multiplied recombinant virus.

When the nucleotide sequence is a nucleotide sequence of the 333rd to 9362nd nucleotides or a nucleotide sequence of the 333rd to 6371st nucleotides, a complete or incomplete non-A, non-B hepatitis virus particle can be obtained.

Furthermore, by using part or whole of the cDNA of Fig. 2(1) through Fig. 2(16) as a template, an RNA or mRNA corresponding thereto can be synthesized by in vitro transcription according to a standard method. For example, an RNA or mRNA corresponding to the entire region of the cDNA of Fig. 2(1) through Fig. 2(16) can synthesized using as a template the entire region of the cDNA which is prepared by digesting plasmid pDM-18 (constructed in Example 2) with restriction enzyme HindIII, followed by in vitro transcription by means of T7 RNA polymerase and cap analog. The thus synthesized RNA or mRNA covers the entire region of NANBV gene, that is, the RNA or mRNA is substantially naked NANBV genome. Therefore, when the mRNA is transfected into animal cells, an infectious NANBV particle can be obtained. The above-mentioned mRNA can be synthesized by means of, for example, a commercially available mRNA Capping Kit (manufactured and sold by Stratagene, U.S.A.) in a conventional manner. With respect to the details of the operating procedure for the synthesis, reference may be made to "Current Protocols in Molecular Biology", 10.17.1 - 10.17.5, published by John Wiley & Sons, 1989). The RNA which can be obtained using part or whole of the cDNA of Fig. 2(1) through Fig. 2(16), is part or whole of the NANBV genome and, therefore, it is useful not only for producing an incomplete NANBV particle, a complete NANBV particle or NANBV antigens but also for studying NANBV and infectious disease caused thereby.

### Step (VII): Purification of an NANBV antigen polypeptide

### Step (VII): Purification of an NANBV antigen polypeptide

The NANBV antigen polypeptide produced in the culture of the transformant or recombinant virus may be purified using an appropriate combination of customary techniques selected from, for example, salting-out; adsorption and desorption using a silica gel, an activated carbon or the like; precipitation by an organic solvent; fractionation by ultracentrifugation; separation by ion exchange chromatography or affinity column chromatography; fractionation by high-performance liquid chromatography or electrophoresis, and the like.

When the NANBV antigen polypeptide is purified from the culture of an E. coli transformant or a yeast transformant, from the viewpoint of effective removal of allergens derived from E. coli and yeast which cause the quality of the final product of the NANBV antigen polypeptide to be markedly lowered, it is preferred that the purification be conducted by, for example, the steps of (1) adsorption and desorption using a silica gel, removal of impurities by adsorption on an activated carbon and (2) fractionation by density gradient centrifugation in this order (Japanese Patent Application Laid-Open Specification No. 63-297). When the NANBV antigen polypeptide is purified from the culture of a recombinant virus, e.g., the culture of a recombinant virus-infected cells, a high purity NANBV antigen polypeptide can be obtained by subjecting a crude solution containing the antigen to purification by ultracentrifugation and density gradient centrifugation repeatedly.

Thus, a solution containing a purified NANBV antigen polypeptide of the present invention is obtained. If desired, the solution may be lyophilized to obtain a purified NANBV antigen polypeptide in a dry form.

The mixed antigen polypeptide of the present invention may be obtained by mixing at least two different types of the NANBV antigen polypeptides obtained by gene expression of at least two different types of cDNAs having different nucleotide sequences.

As described above, the core protein (C protein), matrix protein (M protein) and envelope protein (E protein) of the NANBV are included in the region from the 1st (Met) to 389th (Gly) amino acids shown in Fig. 2(1) through Fig. 2(3). Therefore, the above-mentioned epitopes contained in this region, especially epitopes coded for by nucleotide sequences respectively of the 906th to 953rd nucleotides, of the 1020th to 1046th nucleotides and of the 1194th to 1232nd nucleotides, are extremely useful as antigens. The epitopes may be obtained by polypeptide synthesis. The polypeptide synthesis can be conducted by means of a commercially available polypeptide synthesizer, such as polypeptide synthesizer COUPLER 2100 (manufactured and sold by Du Pont, USA) and polypeptide synthesizer 430A (manufactured and sold by Applied Biosystems, USA). The synthesized antigen polypeptide may be used, for example, for producing a vaccine, a diagnostic reagent and an antibody.

In a further aspect of the present invention, there is provided a replicable recombinant comprising a replicable expression vector selected from a plasmid and an animal virus gene and a deoxyribonucleic acid comprising a nucleotide sequence selected from the group consisting of the first nucleotide sequence comprising at least part of a region from the 1st to 1499th nucleotides or at least part of a region from the 1500th to 9416th nucleotides of the non-A, non-B hepatitis virus nucleotide sequence shown in Fig. 2(1) through Fig. 2(16) hereof and a nucleotide sequence obtained by substituting at least one nucleotide of the first nucleotide sequence in accordance with the degeneracy of the genetic code.

The replicable recombinant can be used not only for producing the NANBV antigen polypeptide of the present invention but also for amplifying the NANBV genomic cDNA of the present invention by replication.

In a preferred embodiment of the present invention with respect to the replicable recombinant for amplifying the NANBV genomic cDNA by replication, the first nucleotide sequence comprises at least six nucleotides of the nucleotide sequence of the 1st to 1499th nucleotides or at least six nucleotides of the nucleotide sequence of the 1500th to 9416th nucleotides.

In a preferred embodiment of the present invention with respect to the replicable recombinant for producing the NANB antigen polypeptide, the first nucleotide sequence comprises at least twelve nucleotides of the nucleotide sequence of the 333rd to 1499th nucleotides or at least twelve nucleotides of the nucleotide sequence of the 1500th to 9362nd nucleotides.

In another preferred embodiemnt of the present invention with respect to the replicable recombinant for producing the NANBV antigen polypeptide, the first nucleotide sequence is selected from the group consisting of a nucleotide sequence of the 333rd to 422nd nucleotides, a nucleotide sequence of the 333rd to 677th nucleotides, a nucleotide sequence of the 333rd to 1499th nucleotides, a nucleotide sequence of the 474th to 563rd nucleotides, a nucleotide sequence of the 678th to 905th nucleotides, a nucleotide sequence of the 906th to 953rd nucleotides, a nucleotide sequence of the 906th to 1499th nucleotides, a nucleotide sequence of the 1020th to 1046th nucleotides, a nucleotide sequence of the 1020th to 1121st nucleotides, a nucleotide sequence of the 1194th to 1232nd nucleotides, a nucleotide sequence of the 1209th to 1322nd nucleotides, a nucleotide sequence of the 1500th to 2519th nucleotides, a nucleotide sequence of the 2520th to 3350th nucleotides, a nucleotide sequence of the 3351st to 5177th nucleotides, a nucleotide sequence of the 4485th to 4574th nucleotides, a nucleotide sequence of the 5178th to 5918th nucleotides, a nucleotide sequence of the 5544th to 5633rd nucleotides, a nucleotide sequence of the 5919th to 6371st nucleotides and a nucleotide sequence of the 6372nd to 9362nd nucleotides.

The purified NANBV antigen polypeptide of the present invention is useful as a diagnostic reagent for detecting NANB hepatitis.

The NANBV antigen polypeptide of the present invention can be formulated into a diagnostic reagent as follows. The purified NANBV antigen polypeptide solution obtained above is dispensed in a vessel, such as a vial and an ampul, and sealed. The antigen polypeptide solution put in a vessel may be lyophilized before the sealing, in the same manner as mentioned above. The amount of the NANBV antigen polypeptide put in a vessel is generally about 1 µg to about 10 mg. Alternatively, the NANBV antigen polypeptide may also be adsorbed on the surface of a customarily employed support, such as a microplate, polyethylene beads, filter paper or a membrane.

The determination of the reactivity of the serum with the NANBV antigen polypeptide may be conducted in substantially the same manner as described in Step (V) mentioned above. That is, the determination of the reactivity may be conducted by a conventional immunoassay method, such as radioimmunoassay (RIA), enzyme-linked immunosorbent assay (ELISA), fluorescent antibody technique (FA), passive haemagglutination (PHA), reversed passive haemagglutination (rPHA) and the like. The amount of the NANBV antigen polypeptide to be used for the above immunoassay is generally from about 0.1 to about 100 mg/ml of serum. Particularly, the amounts of the NANBV antigen polypeptide to be used for RIA, ELISA, FA, PHA and rPHA are generally from 0.1 to 1 mg/ml, from 0.1 to 1 mg/ml, from 1 to 100 mg/ml, from 1 to 50 mg/ml and from 1 to 50 mg/ml, respectively.

The NANBV antigen polypeptide of the present invention may also be used for screening blood for transfusion. The screening method consists in:
a) isolating serum from whole blood;
b) contacting serum of an unknown blood with an isolated NANBV antigen polypeptide comprising at least one amino acid sequence comprising at least part of an amino acid sequence coded for by a deoxyribonucleic acid comprising a coding region from the 333rd to 9362nd nucleotides of the NANBV nucleotide sequence shown in Fig. 2(1) through Fig. 2(16);
c) determining whether the serum reacts with the NANBV antigen polypeptide;
d) classifying the serum as positive or negative to non-A, non-B hepatitis based on the reactivity; and
e) effecting separation of the blood in accordance with the identification.

The contact of serum of an unknown blood with the NANBV antigen polypeptide of the present invention, and the determination of the reactivity of the serum of the blood with the NANBV antigen polypeptide may be conducted in the same manner as mentioned above with respect to the method for diagnosing NANB hepatitis. By the above method, a blood for transfusion free from the NANBV can be selected.

The polyclonal antibody and monoclonal antibody specific for the NANBV antigen polypeptide of the present invention may be used as an agent for removing NANBV from blood for transfusion. That is, NANBV present in blood can efficiently be removed by the polyclonal antibody or the monoclonal antibody by antigen-antibody reaction.

Further, the NANBV antigen polypeptide of the present invention may advantageously be used as an active ingredient of a vaccine for NANB hepatitis. The vaccine for NANB hepatitis may be prepared as follows. The culturing of a transformant containing a recombinant phage or plasmid carrying the cDNA coding for the NANBV antigen polypeptide, or a cell infected with the recombinant virus carrying the cDNA coding for the NANBV antigen polypeptide is conducted in the same manner as described above to thereby produce the NANBV antigen polypeptide in the culture. For detoxifying the NANBV antigen polypeptide in the culture to secure the safety of the antigen polypeptide and for fixing the antigen polypeptide to stabilize the immunogenicity and the antigenicity of the antigen polypeptide, it is preferred to add a conventional inactivating agent to the culture of the transformant or recombinant virus-infected cell, or to a culture medium obtained by removing the transformant cells or the recombinant virus-infected cell. For example, an inactivating agent, such as formalin, may be added in an amount of from 0.0001 to 0.001 v/v%, followed by incubation at 4 to 37 °C for 5 to 90 days. Then, the resultant culture or culture medium is subjected to purification in the same manner as mentioned above. Thus, an original NANB hepatitis vaccine solution containing the purified NANBV antigen polypeptide is obtained.

The original NANB hepatitis vaccine solution is filtered using a microfilter by a standard method to sterilize the solution. The filtrate is diluted with physiological saline so that the protein concentration is about 1 to about 500 µg/ml as measured by the Lowry method. To the resultant solution is then added aluminum hydroxide gel as an adjuvant so that the concentration of the added gel becomes about 0.1 to about 1.0 mg/ml. As an adjuvant, there may also be employed precipitating depositary adjuvants such as calcium phosphate gel, aluminum phosphate gel, aluminum sulfate, alumina and bentonite, and antibody-production inducing adjuvants such as muramyl peptide derivatives, polynucleotides, Krestin® (manufactured and sold by Kureha Chemical Industry Co., Ltd., Japan) and picibanil (both of which are an antineoplastic agent). Further, to the mixture, at least one stabilizing agent may be added. As the stabilizing agent, any commercially available stabilizing agent may be used. Examples of stabilizing agents include gelatin and hydrolysates thereof, albumin, saccharides such as glucose, fructose, galactose, sucrose and lactose, and amino acids such as glycine, alanine, lysine, arginine and glutamine.

Then, the thus obtained NANB hepatitis vaccine solution containing a gel-adsorbed NANBV antigen polypeptide is dispensed into a small vessel, such as an ampul and a vial, and sealed. Thus, there is obtained a purified adsorbed NANB hepatitis vaccine comprising an adsorbed NANBV antigen polypeptide.

The NANB hepatitis vaccine solution thus obtained may be lyophilized to obtain the NANB hepatitis vaccine in a dried form so that the product can be transported to and stored at a place of severe climate, for example, in an area in the tropics. The lyophilization may generally be conducted according to a standard method after the liquid adsorbed NANB hepatitis vaccine is dispensed in a vessel such as a vial and an ampul. After lyophilization, a nitrogen gas is introduced in the vessel containing the dried vaccine, followed by sealing. Incidentally, the quality of the vaccine produced is examined in accordance with "Adsorbed Hepatitis B Vaccine", "Dried Japanese Encephalitis Vaccine", and "Adsorbed Pertussis Vaccine" provided for in Notification No. 159 of the Ministry of Health and Welfare, Japan, "Minimum Requirements for Biological Products".

The NANB hepatitis vaccine may be prepared in the form of a mixed vaccine which contains an adsorbed NANBV antigen polypeptide mentioned above and at least one antigen other than the present NANBV antigen polypeptide. As the antigen other than the present NANBV antigen polypeptide, there may be employed any antigens that are conventionally used as active ingredients of the corresponding vaccines insofar as the side effects and adverse reactions caused by such other antigens and the NANBV antigen polypeptide are not additively or synergistically increased by the use of the NANBV antigen polypeptide and such other antigens in combination and the antigenicities and immunogenicities of the NANBV antigen polypeptide and such other antigens are not reduced by the interference between the NANBV antigen polypeptide and other antigens. The number and the types of the antigens which may be mixed with the NANBV antigen polypeptide are not limited insofar as the side effects and adverse reactions are not increased additively or synergistically and the antigenicity and immunogenicity of each of the NANBV antigen polypeptide and such antigens are not reduced as mentioned above. Generally, two to six types of antigens may be mixed with the NANBV antigen polypeptide. Examples of antigens which may be mixed with the present NANBV antigen polypeptide, include detoxified antigens, inactivated antigens or toxoids which are derived from Japanese encephalitis virus, HFRS (hemorrhagic fever with renal syndrome) virus, influenza virus, parainfluenza virus, hepatitis B virus, dengue fever virus, AIDS virus, Bordetella pertussis, diphtheria bacillus, tetanus bacillus, meningococcus, pneumococcus and the like.

Generally, the vaccine comprising the NANBV antigen polypeptide of the present invention may be contained and sealed in a vial, an ampul or the like. The vaccine of the present invention may generally be administered in the form of a liquid or suspension. In the case where the vaccine is in a dried form, the vaccine is dissolved or suspended in sterilized distilled water before administration, the amount of the distilled water being such that the volume becomes the original volume before being subjected to lyophilization. Generally, the vaccine may be administered subcutaneously. The dose of the vaccine per person may generally be about 0.5 ml. In general, the dose of the vaccine per child may be half as much as that of the vaccine per adult. The vaccine may generally be administered twice at an interval of about one week to one month and then, about half a year later, administered once more.

Further, the NANBV antigen polypeptide may be used for preparing an antibody, such as a polyclonal antibody and a monoclonal antibody, specific for the NANBV antigen polypeptide. For example, a polyclonal antibody specific for the NANBV antigen polypeptide may be prepared by a conventional method as follows. The purified NANBV antigen polypeptide of the present invention is inoculated subcutaneously, intramuscularly, intraperitoneally or intravenously to an animal, such as mouse, guinea pig and rabbit. The inoculation of the NANBV antigen polypeptide is generally conducted several times at intervals of 1 to 4 weeks, to thereby completely immunize the animal. In order to enhance the immunizing effect, a conventional and commercially available adjuvant may be used. Then, blood serum is collected from the immunized animal and an anti-NANBV antigen polypeptide polyclonal antibody is isolated and purified from the blood serum according to a standard method.

On the other hand, a monoclonal antibody specific for the NANBV antigen polypeptide may be prepared by a conventional method as described, for example, in Cell Technology, 1, 23-29 (1982). For example, splenic cells obtained from a mouse immunized with the purified NANBV antigen polypeptide are fused with commercially available mouse myeloma cells by cell fusion technique, to obtain hybridomas. The hybridomas are screened to obtain a hybridoma capable of producing an antibody reactive with the NANBV antigen polypeptide. The obtained hybridoma is cultured in a standard method. From the supernatant of the culture, an anti-NANBV antigen polypeptide monoclonal antibody is isolated and purified by a standard method.

The above-mentioned polyclonal antibody and monoclonal antibody may also be used as a diagnostic reagent for diagnosing NANB hepatitis. The diagnosis of NANB hepatitis using the antibody may be conducted by immunoassay in substantially the same manner as mentioned above with respect to the diagnosis of NANB hepatitis using the NANBV antigen polypeptide. By the use of the polyclonal antibody or the monoclonal antibody, the identification and quantification of the NANBV antigen polypeptide present in a liver tissue and blood can be conducted.

The NANBV genomic cDNA of the present invention can be prepared by digesting the NANBV genomic cDNA clone defined in the present invention with an appropriate restriction enzyme. Also, the NANBV genomic cDNA of the present invention can be prepared by the technique of DNA synthesis in accordance with the nucleotide sequence shown in Fig. 2(1) to Fig. 2(16) of the present application. The preparation of the NANBV genomic cDNA by way of DNA synthesis can be performed by means of a customary DNA synthesizer, such as DNA synthesizer Model 380B (manufactured and sold by Applied Biosystem, U.S.A.) and DNA Synthesizer Model 8700 (manufactured and sold by Biosearch, U.S.A.). The NANBV genomic cDNA of the present invention can be used to conduct the genetic diagnosis of NANBV infection. That is, the NANBV genomic cDNA of the present invention can be used as a primer for polymerase chain reaction (PCR) in the detection of an NANBV gene in the body fluid or cells from a patient. For the diagnosis by polymerase chain reaction, the NANBV genomic cDNA is used in an amount of 10 to 100 ng.

The NANBV genomic cDNA of the present invention may also be used for diagnosing NANB hepatitis by hybridization technique. That is, the NANBV genomic cDNA is labeled with, for example, biotin, alkaline phosphatase, radioisotope ³²P or the like and used as a probe for hybridization. The cDNA to be used for the diagnosis by hybridization technique may be prepared by a standarad method, for example, as follows. The recombinant phage containing the NANBV cDNA obtained in Step (V) mentioned above is digested with an appropriate restriction enzyme to cut off the DNA fragment containing the NANBV cDNA. The obtained DNA fragment is ligated to a commercially available replicable cloning plasmid to obtain a recombinant plasmid containing the DNA fragment. The recombinant plasmid is introduced in a host cell to form a transformant and the transformant is cultured to multiply the recombinant plasmid. The multiplied recombinant plasmid is isolated from the transformant and digested with a restriction enzyme. The resultant digest is subjected to low-melting point agarose gel electrophoresis to isolate and purify the cDNA coding for the NANBV antigen polypeptide. The thus obtained cDNA is labeled with biotin, alkaline phosphatase, radioisotope ³²P or the like. The labeling of the cDNA may be conducted by using a commercially available nick translation kit or multiprime DNA labeling system (manufactured and sold by, for example, Amersham, England; Nippon Gene Co., Ltd., Japan; and the like). The labeled cDNA is put in a vessel having a volume of about 5 to 20 ml, such as a vial or an ampul, and sealed. The amount of the labeled cDNA put in a vessel is generally 1 to 100 µg per vessel. The labeled cDNA may be contained in the vessel in the form of a solution. Alternatively, the labeled cDNA may be contained in the vessel in a lyophilized state. The diagnosis of NANB hepatitis by the use of the labeled cDNA is conducted by a standard hybridization method. That is, plasma, serum or leukocytes obtained from a patient is placed in contact with the labeled cDNA and an RNA hybridized with the labeled cDNA is detected. The detection of the RNA hybridized with the labeled cDNA may be conducted by a standard method. When the cDNA is labeled with an enzyme, the detection is conducted by enzyme immunoassay. When the cDNA is labeled with a radioisotope, the detection is conducted by, for example, scintillation counting.

The NANBV genomic cDNA of the present invention is excellent in reliability and contains the entire region of the open reading frame of the NANBV gene.

The NANBV antigen polypeptide of the present invention is specifically reactive with the NANBV. Therefore, when the NANBV antigen polypeptide is used as a diagnostic reagent, the diagnosis of NANB hepatitis can be conducted easily with high reliability. Further, when the NANBV antigen polypeptide of the present invention is used for screening blood for transfusion, blood which is infected by NANBV can be selected easily with high reliability and removed from blood not infected by NANBV. Therefore, the post-transfusion NANB hepatitis can be prevented.

Further, the NANBV antigen polypeptide of the present invention may advantageously be used as an active ingredient of a vaccine for preventing NANB hepatitis.

Further, by the use of the NANBV antigen polypeptide of the present invention, an antibody, particularly monoclonal antibody, specific for NANBV can easily be prepared. The antibody specific for NANBV can advantageously be used as not only a diagnostic reagent for detecting NANB hepatitis, but also an agent for removing NANBV from blood for transfusion.

Furthermore, it should be noted that the NANBV antigen polypeptide of the present invention is not produced by the infection of an animal with a virus, but produced by gene expression of the DNA coding for the present antigen polypeptide in a host cell. Hence, the possibility of infection during the steps for production of the present antigen polypeptide is substantially eliminated. Also, the production cost can be decreased. Moreover, since all of the materials used in the production process, e.g., medium for the incubation system, are well-known in respect of the composition thereof, purification is facile and an antigen polypeptide product having high purity can be obtained.

The present invention will now be described in detail with reference to the following Examples, which should not be construed to be limiting the scope of the present invention.

### Example 1

### Step 1 (Preparation of a plasma-derived RNA for producing cDNA, which is complementary to NANBV genome RNA)

In order to obtain NANBV from plasma, 4.8 liters of human plasma exhibiting a glutamic-pyruvic transaminase (GPT) activity of 35 IU/litre or more (as measured by the method of Wroblewski, F & J.S. LaDue: Serum glutamic-pyruvic transaminase in cardiac and hepatic disease. Proc. Soc. Exp. Biol. Med., 91:569, 1956) was superposed on a 30 % (w/w) aqueous sucrose solution, and subjected to centrifugation under 48,000 x g at 4 °C and for 13 hours to obtain a precipitate. The precipitate was suspended in an aqueous solution containing 50 mM Tris·HCl (pH 8.0) and 1 mM EDTA, and once more subjected to centrifugation under 250,000 x g at 4 °C and for 3 hours to thereby obtain a precipitate. The obtained precipitate was dissolved in 75 ml of 5.5 M GTC solution containing 5.5 M quanidine thiocyanate, 20 mM sodium citrate (pH 7.0), 0.05 % sarkosyl (sodium lauryl sarcosinate) and 0.1 M 2-mercaptoethanol. The resultant solution was superposed on 16 ml of CsTFA-0.1 M EDTA solution (ρ = 1.51), and subjected to centrifugation under 140,000 x g at 15 °C and for 20 hours to thereby obtain a precipitate of RNA. The supernatant containing proteins and DNA was removed by suction, and the precipitate was dissolved in 200 µl of TE 10 mM Tris·HCl, pH 8.0 and 1 mM EDTA solution. 20 µl of 3 M sodium chloride and ethanol were added to the solution, and allowed to stand still at -70 °C for 90 minutes. The mixture was centrifuged under 12,000 X g at 4 °C and for 30 minutes to obtain a precipitate. The precipitate was dissolved in TE, and sodium chloride and ethanol were added in the same manner as mentioned above. The mixture was allowed to stand still at -70 °C to obtain a precipitate. The precipitate was dissolved in 10 µl of TE to thereby obtain a purified RNA.

### Step 2 (Preparation of a liver-derived RNA for producing a cDNA, which is complementary to NANBV genome RNA)

NANBV genome RNA was prepared from a liver tissue cut off from a NANBV hepatitis patient by the method of Okayama et al. (see H. Okayama, M. Kawaichi, M. Brownstein, F. Lee, T. Yokota, and K. Arai: High-Efficiency Cloning of Full-Length cDNA; Construction and Screening of cDNA Expression Libraries for Mammalian Cells, Methods in Enzymology 154.3-28, 1987).

Illustratively stated, 1 g of liver tissue was cut into small pieces. The small pieces were suspended in 100 ml of 5.5 M GTC solution as used in Step 1, and homogenized by means of a Teflon-glass homogenizer. Subsequently, the introduction of the homogenate into a syringe having #18 needle and the discharge of the homogenate from the syringe through the needle were repeated to thereby mechanically split DNA. The resultant homogenate was centrifuged under 1,500 x g (lower centrifugal force) at 4 °C and for 15 minutes to thereby obtain a supernatant. The supernatant was superposed on CsTFA solution and centrifuged in substantially the same manner as described in Step 1 to thereby obtain a precipitate as an RNA fraction. The thus obtained precipitate was suspended in 0.4 ml of 4 M GTC solution. 10 µl of 1 M acetic acid and 300 µl of ethanol were added to the suspension, and allowed to stand still at a temperature of -20 °C for at least 3 hours to thereby obtain a precipitate of RNA. The precipitate was separated by centrifugation under 12,000 x g at a temperature of 4 °C and for 10 minutes, and dissolved in 1 ml of TE solution. 100 µl of 2 M sodium chloride solution and 3 ml of ethanol were added to the solution, and the mixture was allowed to stand at -20 °C for 3 hours. The resultant precipitate was collected by centrifugation and dissolved in 10 µl of TE to thereby obtain a purified, liver-derived RNA.

### Step 3 (Preparation of a double-stranded cDNA using a cDNA synthesis kit)

A double-stranded cDNA was prepared using a commercially available cDNA synthesis kit (manufactured and sold by Amersham International, England).

Illustratively stated, 0.75 µg of the purified RNA obtained in Step 1 and 2 µl of random hexanucleotide primer and 2 µl of reverse transcriptase taken from the reagents included in the kit were put in a reaction tube. Then, distilled water was added in an amount such that the total volume of the resultant mixture became 20 µl. The mixture was incubated at 42 °C for 40 minutes, thereby preparing a first strand of cDNA. Subsequently, a second strand of cDNA was synthesized while cooling the reaction mixture in ice water, as follows. To 20 µl of the reaction mixture were added 37.5 µl of buffer for second strand synthetic reaction, 1 µl of E. coli ribonuclease H and 6.6 µl of DNA polymerase I, which were taken from the reagents included in the kit, followed by addition of 34.9 µl of distilled water. The mixture was incubated at 12 °C for 60 minutes, 22 °C for 60 minutes and at 70 °C for 10 minutes. Then, the mixture was once more cooled with ice water. 1 µl of T4 DNA polymerase was added, incubated at a temperature of 37 °C for 10 minutes, and 4 µl of 0.25 M EDTA (pH 8.0) was added to thereby terminate the reaction. The reaction mixture was mixed well with a mixture of phenol and chloroform, and centrifuged under 12,000 x g for one minute to thereby separate an aqueous layer. The aqueous layer was again subjected to the same extraction as mentioned above, and an equal amount of chloroform was added. The mixture was agitated well and centrifuged to separate an aqueous layer. Subsequently, an equal amount of 4 M ammonium acetate and a two-fold amount of ethanol were added to the aqueous layer, and the mixture was cooled to -70 °C, thereby obtaining a precipitate of purified double-stranded cDNA. The precipitate was dissolved in 50 µl of 2 M ammonium acetate. To the mixture, 100 µl of ethanol was added, and the resultant mixture was cooled to -70 °C to thereby obtain a precipitate. The precipitate was collected by centrifugation under 12,000 x g for ten minutes. The collected precipitate was dried and then, dissolved in 20 µl of TE.

### Step 4 (Preparation of a double-stranded cDNA by the Polymerase Chain Reaction (PCR) method)

The cDNAs which were prepared by means of a reverse transcriptase using as templates the RNAs prepared in Step 1 and Step 2, were individually amplified by the PCR method (see Saiki, R. K., Gelfand, D. H., Stoffer, S., Scharf, S. J., Higuchi, R., Horn, G. T., Mullis, K. B., and Erlich, H. A., Primer-directed enzymatic amplification of DNA with a thermostable DNA Polymerase, Science 239:487-491, 1988). That is, 5 to 1,000 ng of the RNA was incubated in 20 µl of a reverse transcriptase solution containing 50 mM Tris·HCl (pH 8.3), 40 mM KCl, 6 mM MgCl₂, 1 µM 3'-primer [synthesized oligonucleotide comprised of 25 nucleotides of nucleotide numbers 7949 to 7973 in Fig. 2(14)], 10 mM dNTP, and 0.5 unit of reverse transcriptase (product of New England Bio Lab., U.S.A.) at 37 °C for 30 minutes. To the resultant mixture was added 80 µl of a PCR reaction solution containing 18 mM Tris·HCl (pH 8.3), 48 mM KCl, 1.5 mM MgCl₂, 0.6 µM each of 5'-primer [synthesized oligonucleotide comprised of 25 nucleotides of nucleotide numbers 7612 to 7636 in Fig. 2(13)] and the above-mentioned 3'-primer, 10 mM dNTP and 2.5 units of Taq DNA polymerase (manufactured and sold by Perkin Elmer Cetus Co., Ltd., U.S.A.). The mixture was subjected to incubation at 94 °C for one minute, at 50 °C for 2 minutes and at 72 °C for 3 minutes. This incubation was repeated 40 times. The resultant mixture was subjected to electrophoresis using agarose gel, thereby obtaining amplified cDNA. The amplified cDNA was subjected to phenol treatment, ethanol precipitation and drying. The dried cDNA was dissolved in 10 µl of TE.

### Step 5 (Preparation of a cDNA library using lambda gt11)

Using a commercially available cDNA cloning kit (manufactured and sold by Amersham International, England), a cDNA library was prepared. That is, to 130 ng of cDNA prepared in Step 3 were added 2 µl of L/K buffer, 2 µl of EcoRI adaptor and 2 µl of T4 DNA ligase, which were taken from the reagents included in the cloning kit. Distilled water was added to the solution in an amount such that the total volume of the resultant mixture became 20 µl. The mixture was incubated at a temperature of 15 °C for a period of from 16 to 20 hours, and 2 µl of 0.25 M EDTA was added thereto, to thereby terminate the reaction. Subsequently, the mixture was passed through a size fractionating column included in the kit, thereby removing EcoRI adaptors which were not ligated to the cDNA. To 700 µl of the cDNA having EcoRI adaptor ligated thereto were added 83 µl of L/K buffer and 8 µl of T4 polynucleotidekinase. The mixture was incubated at a temperature of 37 °C for 30 minutes. The resultant mixture was subjected to phenol extraction twice, concentration to 350 to 400 µl by means of butanol and then ethanol precipitation, thereby obtaining a precipitate. The precipitate was dissolved in 5 µl of TE.

Subsequently, in order to insert the cDNA having EcoRI adaptor ligated thereto to the EcoRI site of cloning vector lambda gt11, 1 µl of L/K buffer, 2 µl (1 µg) of lambda gt11 arm DNA and 2 µl of T4 DNA ligase were added to 1 µl (10 ng) of the above-mentioned cDNA having EcoRI adaptor ligated thereto. Distilled water was added to the mixture in an amount such that the total volume of the mixture became 10 µl. The mixture was incubated at a temperature of 15 °C for a period of from 16 to 20 hours. Thus, a recombinant lambda gt11 DNA solution was prepared. Further, a recombinant lambda phage was obtained by in vitro packaging using a commercially available in vitro packaging kit (manufactured and sold by Stratagene Co., Ltd., U.S.A.) including Gigapack II Gold solutions A and B, SM buffer and chloroform. That is, 10 µl of Gigapack II Gold solution A and 15 µl of Gigapack II Gold solution B were added to 4 µl of the above-mentioned recombinant lambda gt11 DNA solution. The mixture was incubated at 22 °C for 2 hours. After the incubation, 470 µl of SM buffer and 10 µl of chloroform were added to thereby obtain a recombinant phage, which was stored at 4 °C.

### Step 6 (Cloning of cDNA using E. coli plasmid pUC19)

Using a commercially available DNA ligation kit (manufactured and sold by Takara Shuzo Co., Ltd., Japan) including solutions A and B, the cDNA was inserted in E. coli plasmid pUC19 (C. Yanishi-Perron, J. Vieira, J. Messing, Gene 33, 103, 1985), and cloned in E. coli. That is, 40 µl of solution A and 10 µl of solution B were added to 5 µl of the cDNA prepared by polymerase chain reaction (PCR) in Step 4 and 5 µl (50 ng) of plasmid pUC19 DNA which had been digested with restriction enzyme SmaI and dephosphorylated. The mixture was incubated at a temperature of 15 °C for 16 hours. E. coli strain JM 109 (see Messing, J., Crea, R., and Seeburg, P.H., Nucleic Acids Res. 9, 309, 1981) was transformed with the above-obtained plasmid DNA according to the calcium chloride method (see Mandel, M. and A. Higa, J. Mol. Biol., 53, 154, 1970). Thus, a transformed E. coli containing the plasmid having the cDNA ligated thereto was obtained.

### Step 7 (Screening of clone having NANBV gene from a cDNA library)

E. coli strain Y 1090 (see Richard A. Young and Ronald W. Davis, Science, 222, 778, 1983) was cultured in 50 ml of LBM medium containing 1 % tryptone, 0.5 % yeast extract, 1 % sodium chloride, 50 µg/ml ampicillin and 0.4 % maltose at a temperature of 37 °C. The E. coli cells in a logarithmic growth phase were suspended in 15 ml of 10 mM magnesium sulfate cooled with ice. The phage solution obtained in Step 5 was diluted with SM buffer containing 0.1 M sodium chloride, 8 mM magnesium sulfate, 50 mM Tris·HCl (pH 7.5) and 0.01 % gelatin. 0.1 ml of the diluted phage solution was mixed with an equal volume of the above-mentioned E. coli cell suspension, and the mixture was incubated at a temperature of 37 °C for 15 minutes. To the mixture was added 4 ml of soft agar medium heated to 45 °C and containing 1 % tryptone, 0.5 % yeast extract, 0.5 % sodium chloride, 0.25 % magnesium sulfate and 0.7 % agar (pH 7.0). The mixture was spread on L-agar plate containing 1 % tryptone, 0.5 % yeast extract, 1 % sodium chloride, 1.5 % agar and 100 µg/ml ampicillin (pH 7.0), and incubated at a temperature of 42 °C for 3 hours. Subsequently, 10 mM IPTG (isopropyl β-D-thiogalactopyranoside) was infiltrated into a nitrocellulose filter, and the nitrocellulose filter was dried and closely contacted with the L-agar plate. The plate was incubated at a temperature of 37 °C for 3 hours. The filter was separated, and washed with TBS buffer three times. The washed filter was immersed in 2 % bovine serum albumin solution, and incubated at room temperature for one hour. 1/20 volume of E. coli lysate solution included in a commercially available immunoscreening kit (manufactured and sold by Amersham International, England) was added to pooled serum from NANB hepatitis patients, and incubated at room temperature for 30 minutes. Thereafter, the serum was diluted to 50-fold with 0.2 % bovine serum albumin-added TBS buffer, and the filter was immersed in the diluted serum solution, and incubated at room temperature for one hour.

The resultant filter was washed four times with a TBS buffer containing 0.05 % Tween 20. The washed filter was immersed in an antibody solution which had been prepared by diluting a peroxidase-labeled anti-human IgG (manufactured and sold by Cappel Co., Ltd., Germany) 1,000-fold for one hour. The filter was washed with the above-mentioned Tween-TBS buffer, and immersed in a solution prepared by adding 0.4 ml of DAB (3,3'-diaminobenzidine tetrahydrochloride) and 15 µl of a 30 % aqueous hydrogen peroxide solution to 50 ml of a TBS buffer, followed by incubation at room temperature for 5 to 30 minutes to allow color development. The resultant filter was completely washed with distilled water to terminate the reaction.

By the above-mentioned procedure, the obtained plaques were purified. As a result, 9 positive clones were isolated, which were, respectively, designated as BK 102, BK 103, BK 105, BK 106, BK 108, BK 109, BK 110, BK 111 and BK 112. All of these clones did not react with serum from a healthy human, but reacted with serum from a patient suffering from NANB hepatitis. See Table 1.

**Table 1**

| Reactivity between the serum obtained from a patient suffering from NANB hepatitis and the recombinant lambda gt11 phage clone | | |
|---|---|---|
| Clone | Serum from healthy person | Serum from NANB hepatitis patient |
| BK 102 | 0/10* | 10/11 |
| BK 103 | 0/10 | 9/11 |
| BK 105 | 0/10 | 11/11 |
| BK 106 | 0/10 | 11/11 |
| BK 108 | 0/10 | 9/11 |
| BK 109 | 0/10 | 9/11 |
| BK 110 | 0/10 | 9/11 |
| BK 111 | 0/10 | 9/11 |
| BK 112 | 0/10 | 10/11 |

| | | |
|---|---|---|
| * the number of positive samples/the number of specimens. | | |

### Step 8 (Determination of the nucleotide sequence of the obtained clones)

Recombinant phage DNAs of clones BK 102 to BK 112 were collected, and the collected DNAs were digested with restriction enzyme EcoRI. Then, cDNA fragments of NANBV were isolated and the isolated cDNAs were individually inserted into plasmid pUC19 at EcoRI site. Using the plasmids, E. coli strain JM 109 was transformed in substantially the same manner as in Step 7. Plasmid DNAs were obtained from the transformed E. coli and purified. The nucleotide sequence of each of the NANBV cDNAs was determined using 7-DEAZA sequencing kit (manufactured and sold by Takara Shuzo Co., Ltd., Japan; see Mizusawa, S., Nishimura, S. and Seela, F. Nucleic Acids Res., 14, 1319, 1986). The relationship between the nucleotide sequences of the obtained cDNA clones is shown in Fig. 1(1).

### Step 9 (Cloning of NANBV cDNA clones from a cDNA library by Genomic Walking)

Probes were prepared by labeling with ³²P-dCTP the cDNA fragments of clone BK 102, clone BK 106 and clone BK 112 which were obtained in Step 8. Using the probes, phage clones containing NANBV cDNAs were obtained by hybridization from the cDNA library of cloning vector lambda gt11 obtained in Step 5 and the above-mentioned probes. That is, plasmid DNAs were prepared from the transformed E. coli with clone BK 102, clone BK 106 and clone BK 112 obtained in Step 8 by the alkali method (see T. Maniatis, E.F. Fritsch, and J. Sambrook: Isolation of Bacteriophage λ and Plasmid DNA: "Molecular Cloning", Cold Spring Harbor Lab., pp 75-96.).

Plasmid DNA of clone BK 102 was digested with restriction enzymes NcoI and HincII, and the resultant 0.7 kb fragments having been on the 5'-terminus side of the DNA were subjected to electrophoresis with agarose gel, and collected. Plasmid DNAs of clone BK 106 and clone BK 112 were digested with restriction enzyme NcoI. In the same manner as mentioned above, 1.1 kb DNA fragments were collected from clone BK 106, and 0.7 kb fragments having been on the 3'-terminus side were collected from clone BK 112. 25 ng to 1 µg of DNA fragments were incubated with [α-³²P]dCTP (3000Ci/mmol; manufactured by Amersham Co., Ltd., England) at a temperature of 37 °C for a period of from 3 to 5 hours, using commercially available DNA labeling kit (manufactured by Nippon Gene Co., Ltd.). Thus, probes for hybridization were prepared.

Subsequently, the cDNA library phage obtained in Step 5 was incubated at a temperature of 42 °C in L-agar medium for 3 hours, as described in Step 7. Further, the phage was incubated at a temperature of 37 °C for 3 hours, and was cooled. A nitrocellulose filter was disposed on the mixture, and was allowed to stand still for a period of from 30 to 60 seconds. Thus, the phage was adsorbed onto the filter.

The filter was subjected to alkali denaturation for a period of from 1 to 5 minutes using an aqueous solution containing 0.5 N sodium hydroxide and 1.5 M sodium chloride and to the neutralization with an aqueous solution containing 0.5 M Tris·HCl (pH 8.0) and 1.5 M sodium chloride for a period of from 1 to 5 minutes. The filter was washed with 2 x SSC solution containing 0.3 M sodium chloride and 0.03 M sodium citrate, air dried, and baked at a temperature of 80 °C for 2 hours.

The filter was incubated at a temperature of 42 °C for 6 hours in a solution for hybridization containing 50 % formamide, 5 x SSC, 5 x Denhart solution, 50 mM phosphoric acid-citric acid buffer (pH 6.5), 100 µg/ml trout sperm DNA and 0.1 % SDS. Then, the filter was immersed in 300 ml of the hybridization solution having 1 ml of the above-mentioned probe of about 4 x 10⁸ cpm/ml added thereto, and incubated at a temperature of 42 °C for 16 to 20 hours. The filter was washed with a 2 x SSC solution containing 0.1% (w/v) SDS and with a 0.1 x SSC solution containing 0.1% (w/v) SDS.

After the washing, the filter was dried, and was subjected to autoradiography. Thus, hybridization positive clones were isolated. As a result, 27 clones being reactive with the probe derived from clone BK 102, 14 clones being reactive with the probe derived from clone BK 106 and 13 clones being reactive with the probe derived from clone BK 112, were obtained, which were respectively designated as BK 114 to BK 169.

The nucleotide sequence of each of clones BK 114 to BK 169 was determined according to the method described in Step 8, followed by mapping for each of the clones. As a result, a map of nucleotide sequence having a length of about 9.5 kb considered to be the approximately total length of the NANBV genome was obtained [see Fig. 1(2)].

Clone BK 157 located on the 5' terminus side was digested with restriction enzyme KpnI to thereby collect a 0.55 kb fragment having been on the 5'-terminus side. Also, clone BK 116 located on the extreme 3'-terminus side was digested with restriction enzymes HpaI and EcoRI to thereby collect a 0.55 kb fragment having been on the 3'-terminus side. A probe labeled with ³²P was prepared in the same manner as described above, and the cDNA library phage obtained in Step 5 was subjected to plaque hybridization. As a result, three new additional clones were separated by the probe derived from the clone BK 157. These new clones were, respectively, designated as clones BK 170, BK 171 and BK 172.

### Step 10 (Analysis of the nucleotide sequence of cDNA)

The entire nucleotide sequence of NANBV gene was determined from the nucleotide sequences of the clones obtained in Steps 8 and 9, and shown in Figs. 2(1) to 2(16). From the Figures, it was assumed that the cloned genomic cDNAs of NANBV were composed of 9416 nucleotides, wherein there was an open reading frame composed of 9030 nucleotides coding for a protein composed of 3010 amino acid residues. The hydrophilicity/hydrophobicity pattern of this protein was similar to that of flavivirus as already reported (see H. Sumiyoshi, C. Mori, I. Fuke et al., Complete Nucleotide Sequence of the Japanese Encephalitis Virus Genome RNA. Virology, 161, 497-510, 1987). Clone BK 157 covers nucleotide numbers 1 to 1962 of Figs. 2(1) to 2(16), clone BK 172 covers nucleotide numbers 5 to 366, clone BK 153 covers nucleotide numbers 338 to 1802, clone BK 138 covers nucleotide numbers 1755 to 5124, clone BK 129 covers nucleotide numbers 4104 to 6973, clone BK 108 covers nucleotide numbers 6886 to 8344 and clone BK 166 covers nucleotide numbers 8082 to 9416. They are preserved as Escherichia coli BK 108 (deposited at Fermentation Research Institute, Japan under accession number FERM BP-2971), BK 129 (deposited at Fermentation Research Institute, Japan under accession number FERM BP-2972), BK 138 (deposited at Fermentation Research Institute, Japan under accession number FERM BP-2973), BK 153 (deposited at Fermentation Research Institute, Japan under accession number FERM BP-2974), BK 157, BK 166 (deposited at Fermentation Research Institute, Japan under accession number FERM BP-2975), and BK 172 (deposited at Fermentation Research Institute, Japan under accession number FERM BP-2976), respectively.

### Step 11 (Production of NANBV-related antigens in E. coli, which antigens are related with the antibody-response accompanying NANBV infection)

Respective cDNAs of clone BK 106, clone BK 111 and clone BK 102 each obtained in Step 8 and cDNA of clone BK 147 obtained in Step 9 were individually inserted into plasmids, and the thus obtained plasmid DNAs were collected by the conventional alkali method. Subsequently, the collected DNA of clone BK 106 was digested with restriction enzymes EcoRI and ClaI to thereby obtain 0.5 µg of a DNA fragment of 0.34 kb in length. The thus obtained DNA fragment was incubated at 37 °C for 60 minutes in a T4 DNA polymerase solution containing 67 mM Tris·HCl (pH 8.8), 6.7 mM magnesium chloride, 16.6 mM ammonium sulfate, 10 mM 2-mercaptoethanol, 6.7 µM EDTA, 0.02 % bovine serum albumin, 0.3 mM dNTP and 2-5 units of T4 DNA polymerase, thereby rendering both terminals blunt. The DNA of clone BK 102 was digested with restriction enzyme BamHI to thereby collect 0.5 µg of a DNA fragment of 0.7 kb in length, and the terminals of the DNA fragment were rendered blunt using T4 DNA polymerase in substantially the same manner as mentioned above. The DNA of clone BK 147 was digested with restriction enzyme Sau3AI to thereby obtain 0.5 µg of a DNA fragment of 1 kb in length and the terminals of the DNA fragment were rendered blunt in the same manner as mentioned above. Also, the DNA of clone BK 111 was digested with restriction enzyme EcoRI to thereby obtain 0.5 µg of a DNA fragment of 1 kb in length, and the terminals of the DNA fragment were rendered blunt in substantially the same manner as mentioned above. Subsequently, the DNA of expression vector pKK 233-2 (Amann, E. and J. Brosius. ATG vector for regulated high-level expression of cloned genes in Escherichia coli. Gene, Vol. 40, 183, 1985) was digested with restriction enzyme HindIII. 2 µg of the resultant DNA was incubated at 37 °C for 20 minutes in a S1 nuclease solution containing 0.3 M sodium chloride, 50 mM sodium acetate (pH 4.5), 1 mM zinc sulfate and 100-200 units of S1 nuclease, and the reaction was terminated by adding 1/10 volume of each of 0.12 M EDTA and 1 M Tris·HCl solution (pH 9.0). Then, phenol extraction was performed, and the vector DNA having blunt terminals was precipitated by ethanol and collected. On the other hand, the DNA of vector pKK 233-2 was digested with restriction enzyme PstI, and the digested DNA was purified by extraction with phenol and precipitation from ethanol. The terminals of 2 µg of the purified vector DNA which had been cleaved by restriction enzyme PstI were rendered blunt by the above-mentioned T4 DNA polymerase reaction. The thus obtained DNA fragments derived from clone BK 106 and clone BK 111 were each cleaved with restriction enzyme HindIII. 0.5 µg of each of the cleaved DNA fragments was mixed with 0.5 µg of a vector DNA having blunt terminals. The DNA fragments derived from clone BK 102 and clone BK 147 were each cleaved with restriction enzyme PstI. 0.5 µg of each of the claved DNA fragments was mixed with 0.5 µg of a vector DNA having terminals thereof rendered blunt. The volume of each of the mixtures was adjusted to 20 µl by adding 2 µl of 10X ligation solution containing 500 mM Tris·HCl (pH 7.5), 100 mM magnesium chloride, 100 mM DTT and 10 mM ATP, 300-400 units of T4 DNA ligase and distilled water. The mixtures were incubated at 14 °C for 12-18 hours, thereby obtaining plasmids, which were respectively designated as pCE-06, pE-11, pB-02 and pS-09. Using each of these plasmid DNAs, E. coli strain JM 109 was transformed in substantially the same manner as de scribed in Step 6, thereby obtaining transformed E. coli.

The transformed E. coli was cultured at 37 °C in LB medium (pH 7.5) containing 1 (w/v) % trypton, 0.5 (w/v) % yeast extract and 1 (w/v) % sodium chloride, and when it was in logarithmic growth phase, 1 mM IPTG (isopropyl-β-D-thiogalactopyranoside) was added to the medium. The culturing was further continued for 3 hours. Then, E. coli cells were collected by centrifugation (10,000 x g for 15 minutes), and the collected cells were lysed in 50 mM Tris·HCl (pH 8.0). The mixture was subjected to ultrasonic treatment (20 KHz, 600 W, 5 minutes), and centrifuged at 10,000 x g for 15 minutes to thereby obtain a supernatant fraction and a precipitate fraction. Each of the fractions was dissolved in a sample buffer containing of 20 (v/v)% glycerol, 0.1 M Tris·HCl (pH 6.8), 2 (w/v)% SDS, 2 (v/v)% 2-mercaptoethanol and 0.02 % BPB, heated at 100 °C for 3 minutes, and subjected to electrophoresis using 0.1 % SDS-7.5 % polyacrylamide gel to separate protein. After the electrophoresis, the protein was transferred to a nitrocellulose filter by trans blot cell (manufactured and sold by BIO·RAD Co., Ltd., U.S.A.). The filter was immersed in 3 % gelatin solution, and allowed to stand still for 60 minutes. The filter was incubated together with serum from a patient suffering from NANB hepatitis, which had been diluted 100-fold, for 2 to 3 hours at room temperature. The filter was washed with distilled water and then with TTBS solution containing 0.02 M Tris·HCl (pH 7.5), 0.5 M sodium chloride and 0.05 (v/v)% Tween 20. Subsequently, the washed filter was immersed in a 2,000 fold-diluted solution of peroxidase-labeled anti-human IgG antibody, and incubated at room temperature for 90 minutes. The filter was washed with distilled water and then with TTBS solution. The washed filter was immersed in a buffer having, added thereto, coloring agent DAB and 30 %, based on substrate, hydrogen peroxide as described in Step 7 for 5 to 30 minutes, following by washing with water, to terminate the reaction.

As a result, as shown in Table 2, all of the antigens produced by the plasmids specifically react with serum from a patient suffering from NANB hepatitis, thereby demonstrating that the proteins produced by the cDNAs inserted in the plasmids are clinically important.

**Table 2**

| Reactivity evaluated by the Western blot method between proteins produced by various plasmids and sera from a patient suffering from NANB hepatitis. | | | | |
|---|---|---|---|---|
| Plasmid | origin of cDNA | Extract | Serum from NANB hepatititis patient | Serum from healthy human |
| pCE-066 | BK 106 | S | ± | - |
| | | P | + | - |
| pE-11-89 | BK 111 | S | ± | - |
| | | P | + | - |
| pB-02-10 | BK 102 | S | + | - |
| | | P | - | - |
| pS-09-07 | BK 147 | S | ± | - |
| | | P | + | - |
| pKK233-3 | - | S | - | - |
| | | P | - | - |

| | | | | |
|---|---|---|---|---|
| S: Supernatant by centrifugation | | | | |
| P: Precipitate by centrifugation | | | | |
| +: positive | | | | |
| ±: slightly positive | | | | |
| -: negative | | | | |

### Step 12 (Purification of NANBV-related antigens produced by E. coli and reactivity thereof with serum from a patient suffering from hepatitis)

The usefulness of the protein produced by the cDNA which was inserted into an expression vector was demonstrated by purifying the protein and using the purified protein as an antigen for ELISA or radioimmunoassay. That is, the lysate of the transformed E. coli which was obtained in Step 11 was subjected to centrifugation at 10,000 x g for 15 minutes, thereby obtaining a supernatant and a precipitate. For example, the precipitate obtained from transformant JM 109/pCE 066 was suspended in a solution of 100 mM Tris·HCl (pH 8.0) and 0.1 % Triton X-100, and the resultant suspension was subjected to ultrasonic treatment at a frequency of 20 KHz (600 W) for one minute, followed by centrifugation at 21,000 x g for 15 minutes, thereby obtaining a precipitate. The precipitate was re-suspended in a solution of 100 mM Tris·HCl (pH 8.0) and 6 M urea, and then subjected to ultrasonic treatment followed by centrifugation.

The resultant supernatant was dialyzed against a solution of 10 mM phosphoric acid buffer (pH 7.5) and 6 M urea to thereby obtain an antigen solution. 20 ml of the antigen solution was passed through a column (21.5 x 250 mm) packed with hydroxyapatite, which had been equilibrated with the above-mentioned buffer, to cause the antigen to be adsorbed onto the packing material. The column was subjected to high speed liquid chromatography (HPLC) wherein elution was performed with the above-mentioned buffer having, added thereto, sodium chloride, the concentration of which was varied from 0 to 2 M with a linear concentration gradient, thereby obtaining a fraction containing an antigen. The obtained fraction was dialyzed against a solution of 50 mM carbonate buffer (pH 9.6) and 0.05 % sodium dodecyl sulfate (SDS).

Further, the supernatant obtained by centrifugation (at 10,000 g for 15 minutes) of the lysate of transformant JM 109/pB-02-10 was treated with 35 % saturated ammonium sulfate, and the obtained precipitate was dissolved in a solution of 50 mM Tris·HCl (pH 8.5) and 100 mM 2-mercaptoethanol. The resultant solution was dialyzed against the above-mentioned buffer. Subsequently, 100 ml of the dialysed solution was passed through a column (22.0 x 200 mm) packed with DEAE cellulose, which had been equilibrated with the above-mentioned buffer, to cause the antigen to be adsorbed onto the packing material. The column was subjected to high performance liquid chromatography wherein elution was performed with a solution of 50 mM Tris·HCl (pH 8.5) and 100 mM 2-mercaptoethanol having, added thereto, sodium chloride, the concentration of which was varied from 0 to 2 M with a linear concentration gradient, thereby pooling a fraction containing the antigen.

The fraction was dialyzed against a solution of 10 mM phosphate buffer (pH 6.8) and 100 mM 2-mercaptoethanol. The dialyzed solution was passed through the column of hydroxyapatite for high performance liquid chromatography, which had been equilibrated by the above-mentioned buffer, to cause the antigen to be adsorbed onto the packing material. The column was subjected to high speed liquid chromatography wherein elution was performed with phosphoric acid, the concentration of which was varied with a linear concentration gradient from 10 to 400 mM, thereby pooling a fraction containing the antigen. The resultant fraction was dialyzed against a solution of 50 mM carbonate buffer (pH 9.6) and 0.05 % SDS.

The precipitate obtained by centrifugation of the lysate of transformant JM 109/pE-11-89 was suspended in 10 mM phosphate buffer (pH 5.5). The suspension was subjected to the above-mentioned ultrasonic treatment for one minute, and then subjected to centrifugation at 21,000 x g for 15 minutes. The resultant precipitate was suspended in a solution of 100 mM carbonate buffer (pH 10.5), 500 mM sodium chloride and 10 mM EDTA. The resultant suspension was again subjected to the ultrasonic treatment for one minute, followed by centrifugation. The resultant supernatant was dialyzed against a solution of 30 mM phosphate buffer and 6 M urea. Subsequently, 20 ml of the dialyzed solution was passed through a CM cellulose column (22 x 200 mm) for high performance liquid chromatography (HPLC), which had been equilibrated with the same buffer as used for the above-mentioned dialysis, to thereby cause the antigen to be adsorbed onto the packing material. The column was subjected to high performance liquid chromatography wherein elution was performed with the above-mentioned buffer having, added thereto, sodium chloride, the concentration of which was varied from 0 to 1.5 M with a linear concentration gradient, obtaining a fraction containing the antigen. The fraction was dialyzed against a solution containing 50 mM carbonate buffer (pH 9.6) and 0.05 % SDS, thereby obtaining a solution containing the antigen.

The antigens prepared above were used as an antigen for ELISA for the clinical diagnosis of infection with non-A, non-B hepatitis virus. The protein concentration of each of the above-mentioned purified antigens was adjusted to 1 µg/ml, and put in each well of Microplate Immulone 600 (manufactured and sold by Greiner, Co., Ltd., Germany) in an amount of 100 µl for use in ELISA, which well was allowed to stand still at 4 °C overnight. The contents of the individual wells were washed well three times with PBS-T buffer containing 10 mM phosphate buffer (pH 7.2), 0.8 % sodium chloride and 0.05 % Tween 20, and sample serum diluted with the PBS-T buffer was added in an amount of 100 µl/well, followed by reaction at 37 °C for one hour. The contents of the individual wells were washed three times with the PBS-T buffer, and a peroxidase-labeled anti-human IgG antibody (manufactured and sold by Cappel Co., Ltd., Germany) which had been diluted 8000-fold with PBS-T buffer containing 10 % fetal calf serum was added in an amount of 100 µl/well. The individual well contents were reacted at 37 °C for one hour, and washed with the PBS-T buffer four times. A substrate coloring agent solution composed of 9 ml of 0.05 M citric acid-phosphate buffer and, contained therein, 0.5 µg of o-phenylenediamine and 20 µl of aqueous hydrogen peroxide, was added in an amount of 100 µl/well. The plate was light shielded, and allowed to stand still at room temperature for 60 minutes. 75 µl of 4 N sulfuric acid was added to each of the wells, and the absorbance at 490 nm was determined. The results are shown in Table 3. As apparent from the table, all of the antigens derived from the transformants specifically react with the serum from NANB hepatitis patient, thereby attesting to the usefulness in clinical diagnosis of the antigens produced by the transofrmants.

**Table 3**

| Reactivity in ELISA between the purified antigens from various transformed Escherichia coli and the serum from NANB hepatitis patient | | | | | |
|---|---|---|---|---|---|
| origin of antigen (transformed Escherichia coli) | Serum from blood transfused patient of hepatitis | | | | |
| | acute | chronic | hepatocirrhosis | hepatoma | healthy human serum |
| JM109/pCE-066 | 2/3* | 7/8 | 3/4 | 3/3 | 0/10 |
| JM109/pB-02-10 | 2/3 | 8/8 | 4/4 | 3/3 | 0/10 |
| JM109/pE-11-89 | 2/3 | 8/8 | 2/4 | 3/3 | 0/10 |

| | | | | | |
|---|---|---|---|---|---|
| *: the number of positive samples/the number of samples examined | | | | | |

The same results as shown in Table 3 were also obtained by radioimmunoassay using the above-mentioned antigens. That is, a polystyrene ball of 1/4 inch in diameter (manufactured and sold by Pesel Co., Ltd., Germany) was put in 0.2 ml of each of the above-mentioned purified antigen solutions of 1 µg/ml in concentration, and allowed to stand still at 4 °C overnight. Then, the polystyrene ball was washed five times with the same PBS-T buffer as used in the above-mentioned ELISA, and a sample serum diluted 20 to 2500-fold with the PBS-T buffer was added in an amount of 200 µl/ball. Reaction was performed at 37 °C for 60 min. The polystyrene ball was washed five times with the PBS-T buffer, and ¹²⁵I-labeled anti-human IgG antibody was added in an amount of 200 µl/ball. Reaction was performed at 37 °C for one hour and the ball was washed five times with the PBS-T buffer. The cpm of ¹²⁵I bound to the polystyrene ball was measured, thereby obtaining the same results as shown in Table 3. Thus, the usefulness of the purified antigens obtained above in the clinical diagnosis of infection with NANB hepatitis virus, was demonstrated.

### Application Example 1

### (Assay of the reactivity of synthetic polypeptide)

The antibody molecule reacts with a specific region structure known as "epitope" which exists on the antigen molecule, to thereby form a bonding therebetween. Such a specific region can be found in the hydrophilic region of the antigen molecule. The antigen polypeptide having such a specific region is believed to be useful for easily preparing a valuable clinical diagnostic reagent with high reaction specificity. The NANBV epitope is presumed from the hydrophilicity/hydrophobicity pattern of the amino acid sequence coded for by the NANBV genomic cDNA shown in Figs. 2(1) to 2(16) Namely, polypeptides BKP-106-1, BKP-106-2, BKP-102-1 and BKP-147-1 were prepared, which were respectively comprised of amino acid residues coded for by nucleotide numbers 333 to 422 shown in Fig. 2(1), nucleotide numbers 474 to 563 shown in Fig. 2(1) through Fig. 2(2), nucleotide numbers 4485 to 4574 shown in Fig. 2(8), and nucleotide numbers 5544 to 5633 shown in Fig. 2(10). The concentration of each of the prepared polypeptides was adjusted to 1 µg/ml, applied to a microplate for ELISA according to the same method as described in Step 12 to thereby form a solid phase, and examined with respect to the reactivity thereof with the serum from NANB hepatitis patient by the method of ELISA. The results are shown in Table 4. As apparent from the table, all of the prepared polypeptides specifically reacted with the serum from NANB hepatitis patient, thereby demonstrating the importance in clinical diagnosis of the particular regions of nucleotide sequences described above.

**Table 4**

| Reactivity of synthetic polypeptides with the serum from NANB hepatitis patient | | | |
|---|---|---|---|
| synthetic polypeptides | serum from NANB hepatitis patient | | |
| | acute | chronic | healthy human |
| BKP-106-1 | 2/5 | 5/5 | 0/5 |
| BKP-106-2 | 2/5 | 5/5 | 0/5 |
| BKP-102-1 | 3/5 | 5/5 | 0/5 |
| BKP-147-1 | 2/5 | 5/5 | 0/5 |

Moreover, presuming the epitopes of the envelop protein of NANBV, three types of proteins were prepared. That is, proteins coded for by nucleotide numbers 906 to 953 shown in Fig.2(2), nucleotide numbers 1020 to 1046 shown in Fig. 2(2) and nucleotide numbers 1194 to 1232 shown in Fig. 2(2) through Fig. 2(3), were prepared. All of the thus prepared polypeptides correspond to the regions of the envelop where antigenic variation is believed to occur depending on the type of the NANBV strain, and the reactivity thereof in ELISA with the serum from a NANB hepatitis patient was confirmed. These attest to the importance and usefulness of the above-mentioned proteins in immunological survey, clinical diagnosis and vaccination.

### Application Example 2

### [Detection of NANBV nucleic acid according to PCR (Polymerase Chain Reaction) method]

For preventing NANB hepatitis caused by blood transfusion, it is important to determine whether or not any NANBV infection exists in the blood supplied for transfusion. Further, for diagnosing hepatitis, it is extremely clinically important to study whether or not any NANBV infection exists in liver tissue. The NANBV cDNA of the present invention can be advantageously used for producing a primer for polymerase chain reaction (PCR) useful for detecting NANB hepatitis. That is, as described in Step 1, the purification of RNA and the preparation of cDNA were performed from 1 ml of serum. Likewise, cDNA was prepared from liver cells as described in Step 2. Subsequently, as described in Step 4, PCR and electrophoresis were conducted. According to the customary procedure, whether or not the amplified cDNA was derived from NANBV, was investigated by Southern hybridization using ³²P-labeled probe prepared from the cDNA derived from NANBV cDNA clone BK 108.

The results are shown in Table 5. From the table, it is apparent that the NANBV nucleic acid in serum can be detected and the serum infection with NANBV can be diagnosed by the use of the primer prepared from the nucleotide sequence of the NANBV cDNA obtained according to the present invention and the fragment of cloned NANBV cDNA as a probe.

**Table 5**

| Detection of NANBV nucleic acid by PCR | | | |
|---|---|---|---|
| sample | | antibody against NANBV | PCR |
| serum from chronic hepatitis patient | | | |
| NANB | 1 | + | + |
| | 2 | + | + |
| HBV carrier | 1 | - | - |
| | 2 | - | - |
| healthy human | 1 | - | - |
| | 2 | - | - |
| excised liver from NANB hepatoma-1 | | + | |
| cancerous site | | | + |
| non-cancerous site | | | + |
| excised liver from NANB hepatoma-2 | | + | |
| cancerous site | | | + |
| non-cancerous site | | | + |

### Example 2

### (Construction of the plasmids for the expression of the entire coding region of the NANBV genomic cDNA and the expression of the NANBV genomic cDNA in E. coli)

cDNA was isolated from each of clones BK112, BK146, BK147, BK157 and BK166 obtained in Steps 8 and 9, and plasmids for the expression of the entire coding region of the NANBV gene were prepared in accordance with the method described in Step 11, as follows.

The plasmid DNA of clone BK157 was digested with restriction enzyme BamHI and subjected to agarose gel electrophoresis to thereby obtain a DNA fragment of 1.3 kb in length. The DNA fragment was inserted in plasmid pUC19 at its BamHI site to thereby obtain plasmid pBam157. The plasmid pBam157 was digested with restriction enzymes XbaI and NcoI to thereby obtain a DNA fragment of about 3.9 kb in length. Separately, an oligonucleotide of 93bp (having 4 nucleotides deleted from the 3'-terminus side) was synthesized by ligating the sequence of the promoter region of 20bp (TAATACGACTCACTATAGGG) of bacteriophage T7RNA polymerase having, attached thereto, XbaI linker sequence to the sequence of nucleotide numbers 1 to 73 shown in Fig. 2(1) having, attached thereto, NcoI linker sequence. The thus obtained oligonucleotide was ligated to the above-mentioned DNA fragment of 3.9 kb, thereby obtaining plasmid pDM-16. Then, pDM-16 was digested with restriction enzymes ClaI and EcoRI to obtain a DNA fragment of about 3.5 kb. Separately, the DNA of clone BK146 was digested with restriction enzymes ClaI and EcoRI to obtain a DNA fragment of 4.1 kb in length. The above-mentioned DNA fragment of about 3.5 kb was ligated to the thus obtained DNA fragment of 4.1 kb, to thereby obtain plasmid pDM-9. Then, plasmid pDM-9 was digested with SacII, thereby obtaining a DNA fragment of 2.7 kb and a DNA fragment of 4.9 kb. The DNA fragment of 4.9 kb was ligated at its SacII site with T4DNA ligase and then digested with BamII and EcoRI, thereby obtaining a DNA fragment of about 7.5 kb. Separately, the DNA of clone BK147 was digested with BamI and EcoRI, thereby obtaining a DNA fragment of about 2 kb. The thus obtained DNA fragment was ligated to the above-mentioned DNA fragment of 7.5 kb to thereby obtain plasmid pBE147. Plasmid pBE147 was digested with SacII. The above-mentioned DNA fragment of 2.7 kb derived from pDM-9 was inserted into the SacII-digested pBE147, thereby obtaining plasmid pDM-B3. Plasmid pDM-B3 was digested with XbaI and EcoRI to thereby obtain a DNA fragment of 6.7 kb.

The DNA of clone BK166 was digested with BamHI to obtain a DNA fragment of 1.3 kb. This fragment was inserted in pUC19 at its BamHI site to obtain pBam166. pBam166 was digested with NdeI and HindIII to thereby obtain a DNA fragment of 2.8 kb. The DNA of clone BK112 was digested with EcoRI and NdeI to obtain a DNA fragment of about 1.6 kb. pUC19 was digested with EcoRI and HindIII to obtain a DNA fragment of about 2.6 kb. The above-obtained three types of DNA fragments were mixed and reacted with T4 DNA ligase to thereby obtain plasmid pEN112 in which these fragments were ligated together at their EcoRI site, NdeI site and HindIII site. Plasmid pEN112 was digested with EcoRI and XbaI to obtain a DNA fragment of 2.7 kb. pSac4629 was digested with EcoRI and XbaI to obtain a fragment of 6.7 kb. The above-mentioned fragment of 2.7 kb was ligated to the fragment of 6.7 kb, and the resultant ligated DNA fragment was inserted in pUC19 at its XbaI site, thereby obtaining plasmids pDM-22 and pDM-18. Plasmid pDM-18 can be used for the transformation of an animal cell or the like so that the cell can produce an NANBV antigen polypeptide. The transformation can also be performed using an RNA prepared by transcripting pDM-18 by means of in vitro Transcription Kit (manufactured and sold by Berlinger Manhein Yamanouchi, Japan). Plasmid pDM-22, in which the cDNA and pDM-18 were inserted in opposite orientations, was digested with HindIII and ClaI to obtain a DNA fragment of about 9 kb.

The DNA of clone BK106 was digested with BamHI to obtain a DNA fragment of about 1.0 kb. This fragment was inserted in plasmid pUC19 at its BamHI site to obtain plasmid pBam106. The thus obtained plasmid DNA was digested with NcoI, and the sticky terminus was rendered blunt with Mang Bean nuclease (manufactured and sold by Takara Shuzo Co., Ltd., Japan). The resultant plasmid was further digested with XbaI, thereby obtaining a fragment of about 3.6 kb. A synthetic oligonucleotide prepared by ligating the sequence of nucleotide numbers 333 to 372 shown in Fig. 2(1) to the downstream of XbaI linker, was ligated to this fragment to thereby obtain plasmid pXb106. Plasmid pXb106 was digested with HindIII and ClaI to obtain a DNA fragment of 0.4 kb. This fragment was ligated to the above-mentioned fragment of about 9 kb derived from plasmid pDM-22 to thereby obtain plasmid pORF-24. Plasmid pORF-24 was digested with XbaI to obtain a DNA fragment of about 9.0 kb. This fragment was ligated to an expression vector (see F. William Studier and B.A.Moffatt, J. Mol. Biol., 189, 113, 1986) having, ligated thereto, T7 RNA polymerase gene promotor, thereby obtaining expression plasmid pJF-22. Using this plasmid DNA, Escherichia coli strain JM109 (DE3) (manufactured and sold by Promega Co., U.S.A.) was transformed in substantially the same manner as in Step 6, thereby obtaining transformant JM109 (DE3)/pJF-22.

Transformant E. coli JM109 (DE3)/pJF-22 was subjected to the subsequent procedure as described in Example 11. That is, the E. coli was cultured on LB culture medium, and then 0.5 mM IPTG was added thereto, followed by further culturing for 3 hours. After that period, the cultured cells were collected and heated in a buffer containing 2 % SDS and 2 % 2-mercaptoethanol at 100 °C for 3 minutes. The resultant cells were subjected to Western blotting analysis to identify the obtained protein. In the Western blotting analysis, use was made of specific antisera obtained by purifying the NANBV-related antigen prepared from the transformant obtained in Step 11 and immunizing guinea pigs therewith. As a result of the Western blotting analysis, it was found that the protein produced by transformant JM109 (DE3)/pJF-22 reacted with all of the antisera. Thus, it was demonstrated that in this transformant, the expression was attained of the entire coding region of the NANBV gene from the 5'-end of the genome coding for the core protein to the 3'-end of the genome coding for the non-structural protein NS5. From the results, it is apparent that this transformant can provide an antigen which is extremely useful for producing not only a diagnostic agent for NANBV infection but also a vaccine for NANBV.

### Example 3

### (Production of NANBV particles by expression of NANBV genomic cDNA in animal cells)

Plasmid pORF-24 obtained in Example 2 was partially digested with XbaI and subjected to low melting point agarose gel electrophoresis to obtain DNA fragments of about 9 kb in length. The thus obtained DNA fragments are digested with Bal31, and XhoI linker is ligated thereto. The DNA fragments each having this linker ligated thereto are individually inserted into plasmid pMAM-neo (available from Clontech, U.S.A.) which had been cleaved with NheI and XhoI, to thereby construct expression plasmids. From these expression plasmids was selected expression plasmid pDEL-NS5 containing no NS5 region through the confirmation of the deficiency of the NS5 region coding for RNA polymerase by a conventional sequencing method using a synthesized primer. The thus selected expression plasmid was transfected into cells of human hepatocyte Chan Liver (ATCC CCL 13) and Chimpanzee hepatocyte (purchased from Dainippon Pharmaceutical Company, Ltd., Japan) individually by a calcium phosphate method ("Molecular Cloning", 16.33-16.39, Cold Spring Harbor Laboratory, 1989). The hepatocyte cells having plasmid pDEL-NS5 introduced thereto was rendered resistant to aminoglycoside antibiotic G418. Utilizing this resistance as a criterion, transformants were selected, followed by cloning. With respect to the polypeptide produced by the cell culture of each of the above-obtained clones, determination of antigenicity was conducted by fluorescent antibody technique using specific antisera used in Example 2. As a result, it was found that the polypeptide produced in the G418 resistant cell clone reacted with all of the antisera except the antiserum of guinea pig immunized with the polypeptide which was produced by expression of plasmid pE-11-89 obtained in Step 11 of Example 1. This fact means that the coding region of NANBV gene exclusive of NS5 region, namely, the region from core protein through NS4 was expressed. Further, it is believed that these antigen peptides are partially agglutinated to form virus-like particles, that is, RNA-deficient NANBV particles.

## Claims

1. An isolated deoxyribonucleic acid comprising at least one nucleotide sequence selected from the following nucleotide sequences and respective complementary sequences to said nucleotide sequences:
a nucleotide sequence of the 333rd to 677th nucleotides,
a nucleotide sequence of the 333rd to 1499th nucleotides,
a nucleotide sequence of the 333rd to 6371st nucleotides,
a nucleotide sequence of the 333rd to 9362nd nucleotides,
a nucleotide sequence of the 474th to 563rd nucleotides,
a nucleotide sequence of the 678th to 905th nucleotides,
a nucleotide sequence of the 906th to 953rd nucleotides,
a nucleotide sequence of the 906th to 1499th nucleotides,
a nucleotide sequence of the 1020th to 1046th nucleotides,
a nucleotide sequence of the 1020th to 1121st nucleotides,
a nucleotide sequence of the 1194th to 1232nd nucleotides,
a nucleotide sequence of the 1209th to 1322nd nucleotides,
a nucleotide sequence of the 1500th to 2519th nucleotides,
a nucleotide sequence of the 2520th to 3350th nucleotides,
a nucleotide sequence of the 3351st to 5177th nucleotides,
a nucleotide sequence of the 4485th to 4574th nucleotides,
a nucleotide sequence of the 5178th to 5918th nucleotides,
a nucleotide sequence of the 5544th to 5633rd nucleotides,
a nucleotide sequence of the 5919th to 6371st nucleotides and
a nucleotide sequence of the 6372nd to 9362nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or comprising a nucleotide sequence obtained by substituting at least one nucleotide of said nucleotide sequence in accordance with the degeneracy of the genetic code.

2. An isolated antigen polypeptide comprising at least one amino acid sequence coded for by a nucleotide sequence selected from the following nucleotide sequences:
a nucleotide sequence of the 333rd to 677th nucleotides,
a nucleotide sequence of the 333rd to 1499th nucleotides,
a nucleotide sequence of the 333rd to 6371st nucleotides,
a nucleotide sequence of the 474th to 563rd nucleotides,
a nucleotide sequence of the 678th to 905th nucleotides,
a nucleotide sequence of the 906th to 953rd nucleotides,
a nucleotide sequence of the 906th to 1499th nucleotides,
a nucleotide sequence of the 1020th to 1046th nucleotides,
a nucleotide sequence of the 1020th to 1121st nucleotides,
a nucleotide sequence of the 1194th to 1232nd nucleotides,
a nucleotide sequence of the 1209th to 1322nd nucleotides,
a nucleotide sequence of the 1500th to 2519th nucleotides,
a nucleotide sequence of the 2520th to 3350th nucleotides,
a nucleotide sequence of the 3351st to 5177th nucleotides,
a nucleotide sequence of the 4485th to 4574th nucleotides,
a nucleotide sequence of the 5178th to 5918th nucleotides,
a nucleotide sequence of the 5544th to 5633rd nucleotides,
a nucleotide sequence of the 5919th to 6371st nucleotides,
a nucleotide sequence of the 6372nd to 9362nd nucleotides and
a nucleotide sequence from the 333rd to 9362nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof.

3. A method for producing a non-A, non-B hepatitis virus antigen polypeptide, which comprises:
(a) inserting a deoxyribonucleic acid into a replicable expression vector selected from a replicable plasmid vector and a replicable animal virus genome vector to obtain a replicable recombinant DNA molecule comprising said replicable plasmid vector and said deoxyribonucleic acid inserted therein when said replicable expression vector is a replicable plasmid vector, or to obtain a replicable recombinant animal virus genome molecule comprising said animal virus genome vector and said deoxyribonucleic acid inserted therein when said replicable expression vector is a replicable animal virus genome vector,
said deoxyribonucleic acid comprising a nucleotide sequence selected from the following nucleotide sequences:
a nucleotide sequence of the 333rd to 677th nucleotides,
a nucleotide sequence of the 333rd to 1499th nucleotides,
a nucleotide sequence of the 333rd to 6371th nucleotides,
a nucleotide sequence of the 474th to 563rd nucleotides,
a nucleotide sequence of the 678th to 905th nucleotides,
a nucleotide sequence of the 906th to 953rd nucleotides,
a nucleotide sequence of the 906th to 1499th nucleotides,
a nucleotide sequence of the 1020th to 1046th nucleotides,
a nucleotide sequence of the 1020th to 1121st nucleotides,
a nucleotide sequence of the 1194th to 1232nd nucleotides,
a nucleotide sequence of the 1209th to 1322nd nucleotides,
a nucleotide sequence of the 1500th to 2519th nucleotides,
a nucleotide sequence of the 2520th to 3350th nucleotides,
a nucleotide sequence of the 3351st to 5177th nucleotides,
a nucleotide sequence of the 4485th to 4574th nucleotides,
a nucleotide sequence of the 5178th to 5918th nucleotides,
a nucleotide sequence of the 5544th to 5633rd nucleotides,
a nucleotide sequence of the 5919th to 6371st nucleotides,
a nucleotide sequence of the 6372nd to 9362nd nucleotides and
a nucleotide sequence of the 333rd to 9362nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or comprising a nucleotide sequence obtained by substituting at least one nucleotide of said nucleotide sequence in accordance with the degeneracy of the genetic code;
(b) transfecting cells of a microorganism or eukaryotic cell culture with said recombinant DNA molecule when said replicable expression vector used in step (a) is a replicable expression plasmid vector, to thereby form a transformant, followed by selection of said transformant from parent cells of the microorganism or eukaryotic cell culture;
(c) culturing said transformant obtained in step (b) to thereby express said deoxyribonucleic acid and produce a non-A, non-B hepatitis virus antigen peptide, or culturing said replicable recombinant animal virus genome molecule obtained in step (a) to thereby express said deoxyribonucleic acid and said animal virus genome and produce a non-A, non-B hepatitis virus antigen polypeptide and an animal virus; and
(d) isolating said non-A, non-B hepatitis virus antigen polypeptide.

4. The method according to claim 3, wherein said nucleotide sequence is a nucleotide sequence of the 333rd to 9362nd nucleotides or a nucleotide sequence of the 333rd to 6371st nucleotides.

5. A replicable recombinant DNA molecule comprising a replicable plasmid vector and a deoxyribonucleic acid comprising at least one nucleotide sequence selected from the following nucleotide sequences and respective complementary sequences to said nucleotide sequences:
a nucleotide sequence of the 333rd to 677th nucleotides,
a nucleotide sequence of the 333rd to 1499th nucleotides,
a nucleotide sequence of the 333rd to 6371th nucleotides,
a nucleotide sequence of the 474th to 563rd nucleotides,
a nucleotide sequence of the 678th to 905th nucleotides,
a nucleotide sequence of the 906th to 953rd nucleotides,
a nucleotide sequence of the 906th to 1499th nucleotides,
a nucleotide sequence of the 1020th to 1046th nucleotides,
a nucleotide sequence of the 1020th to 1121st nucleotides,
a nucleotide sequence of the 1194th to 1232nd nucleotides,
a nucleotide sequence of the 1209th to 1322nd nucleotides,
a nucleotide sequence of the 1500th to 2519th nucleotides,
a nucleotide sequence of the 2520th to 3350th nucleotides,
a nucleotide sequence of the 3351st to 5177th nucleotides,
a nucleotide sequence of the 4485th to 4574th nucleotides,
a nucleotide sequence of the 5178th to 5918th nucleotides,
a nucleotide sequence of the 5544th to 5633rd nucleotides,
a nucleotide sequence of the 5919th to 6371st nucleotides,
a nucleotide sequence of the 6372nd to 9362nd nucleotides and
a nucleotide sequence of the 333rd to 9362nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or comprising a nucleotide sequence obtained by substituting at least one nucleotide of said nucleotide sequence in accordance with the degeneracy of the genetic code.

6. A replicable recombinant animal virus genome molecule comprising an animal virus genome vector and a deoxyribonucleic acid comprising at least one nucleotide sequence selected from the following nucleotide sequences and respective complementary sequences to said nucleotide sequences:
a nucleotide sequence of the 333rd to 677th nucleotides,
a nucleotide sequence of the 333rd to 1499th nucleotides,
a nucleotide sequence of the 333rd to 6371st nucleotides,
a nucleotide sequence of the 474th to 563rd nucleotides,
a nucleotide sequence of the 678th to 905th nucleotides,
a nucleotide sequence of the 906th to 953rd nucleotides,
a nucleotide sequence of the 906th to 1499th nucleotides,
a nucleotide sequence of the 1020th to 1046th nucleotides,
a nucleotide sequence of the 1020th to 1121st nucleotides,
a nucleotide sequence of the 1194th to 1232nd nucleotides,
a nucleotide sequence of the 1209th to 1322nd nucleotides,
a nucleotide sequence of the 1500th to 2519th nucleotides,
a nucleotide sequence of the 2520th to 3350th nucleotides,
a nucleotide sequence of the 3351st to 5177th nucleotides,
a nucleotide sequence of the 4485th to 4574th nucleotides,
a nucleotide sequence of the 5178th to 5918th nucleotides,
a nucleotide sequence of the 5544th to 5633rd nucleotides,
a nucleotide sequence of the 5919th to 6371st nucleotides,
a nucleotide sequence of the 6372nd to 9362nd nucleotides and
a nucleotide sequence of the 333rd to 9362nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or comprising a nucleotide sequence obtained by substituting at least one nucleotide of said nucleotide sequence in accordance with the degeneracy of the genetic code.

7. A diagnostic reagent for the detection of non-A, non-B hepatitis by hybridization or polymerase chain reaction, comprising an effective amount, for the hybridization or polymerase chain reaction, of an isolated deoxyribonucleic acid comprising at least one nucleotide sequence selected from the following nucleotide sequences and respective complementary sequences to said nucleotide sequences:
a nucleotide sequence of the 333rd to 677th nucleotides,
a nucleotide sequence of the 333rd to 1499th nucleotides,
a nucleotide sequence of the 333rd to 6371st nucleotides,
a nucleotide sequence of the 333rd to 9362nd nucleotides,
a nucleotide sequence of the 474th to 563rd nucleotides,
a nucleotide sequence of the 678th to 905th nucleotides,
a nucleotide sequence of the 906th to 953rd nucleotides,
a nucleotide sequence of the 906th to 1499th nucleotides,
a nucleotide sequence of the 1020th to 1046th nucleotides,
a nucleotide sequence of the 1020th to 1121st nucleotides,
a nucleotide sequence of the 1194th to 1232nd nucleotides,
a nucleotide sequence of the 1209th to 1322nd nucleotides,
a nucleotide sequence of the 1500th to 2519th nucleotides,
a nucleotide sequence of the 2520th to 3350th nucleotides,
a nucleotide sequence of the 3351st to 5177th nucleotides,
a nucleotide sequence of the 4485th to 4574th nucleotides,
a nucleotide sequence of the 5178th to 5918th nucleotides,
a nucleotide sequence of the 5544th to 5633rd nucleotides,
a nucleotide sequence of the 5919th to 6371st nucleotides and
a nucleotide sequence of the 6372nd to 9362nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or comprising a nucleotide sequence obtained by substituting at least one nucleotide of said nucleotide sequence in accordance with the degeneracy of the genetic code.

8. A diagnostic reagent for the detection of non-A, non-B hepatitis by antigen-antibody reaction, comprising an effective amount, for the antigen-antibody reaction, of an isolated antigen polypeptide comprising at least one amino acid sequence coded for by a nucleotide sequence selected from the following nucleotide sequences:
a nucleotide sequence of the 333rd to 677th nucleotides,
a nucleotide sequence of the 333rd to 1499th nucleotides,
a nucleotide sequence of the 333rd to 6371st nucleotides,
a nucleotide sequence of the 474th to 563rd nucleotides,
a nucleotide sequence of the 678th to 905th nucleotides,
a nucleotide sequence of the 906th to 953rd nucleotides,
a nucleotide sequence of the 906th to 1499th nucleotides,
a nucleotide sequence of the 1020th to 1046th nucleotides,
a nucleotide sequence of the 1020th to 1121st nucleotides,
a nucleotide sequence of the 1194th to 1232nd nucleotides,
a nucleotide sequence of the 1209th to 1322nd nucleotides,
a nucleotide sequence of the 1500th to 2519th nucleotides,
a nucleotide sequence of the 2520th to 3350th nucleotides,
a nucleotide sequence of the 3351st to 5177th nucleotides,
a nucleotide sequence of the 4485th to 4574th nucleotides,
a nucleotide sequence of the 5178th to 5918th nucleotides,
a nucleotide sequence of the 5544th to 5633rd nucleotides,
a nucleotide sequence of the 5919th to 6371st nucleotides,
a nucleotide sequence of the 6372nd to 9362nd nucleotides and
a nucleotide sequence from the 333rd to 9362nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof.

9. A vaccine for non-A, non-B hepatitis, comprising an effective immunogenic amount of an isolated antigen polypeptide comprising at least one amino acid sequence coded for by a nucleotide sequence selected from the following nucleotide sequences:
a nucleotide sequence of the 333rd to 677th nucleotides,
a nucleotide sequence of the 333rd to 1499th nucleotides,
a nucleotide sequence of the 333rd to 6371st nucleotides,
a nucleotide sequence of the 474th to 563rd nucleotides,
a nucleotide sequence of the 678th to 905th nucleotides,
a nucleotide sequence of the 906th to 953rd nucleotides,
a nucleotide sequence of the 906th to 1499th nucleotides,
a nucleotide sequence of the 1020th to 1046th nucleotides,
a nucleotide sequence of the 1020th to 1121st nucleotides,
a nucleotide sequence of the 1194th to 1232nd nucleotides,
a nucleotide sequence of the 1209th to 1322nd nucleotides, and
a nucleotide sequence from the 333rd to 9362nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof; and at least one pharmaceutically acceptable carrier, diluent or excipient.

10. An isolated RNA synthesized using as a template a deoxyribonucleic acid comprising at least one nucleotide sequence selected from the following nucleotide sequences and respective complementary sequences to said nucleotide sequences:
a nucleotide sequence of the 333rd to 677th nucleotides,
a nucleotide sequence of the 333rd to 1499th nucleotides,
a nucleotide sequence of the 333rd to 6371st nucleotides,
a nucleotide sequence of the 333rd to 9362nd nucleotides,
a nucleotide sequence of the 474th to 563rd nucleotides,
a nucleotide sequence of the 678th to 905th nucleotides,
a nucleotide sequence of the 906th to 953rd nucleotides,
a nucleotide sequence of the 906th to 1499th nucleotides,
a nucleotide sequence of the 1020th to 1046th nucleotides,
a nucleotide sequence of the 1020th to 1121st nucleotides,
a nucleotide sequence of the 1194th to 1232nd nucleotides,
a nucleotide sequence of the 1209th to 1322nd nucleotides,
a nucleotide sequence of the 1500th to 2519th nucleotides,
a nucleotide sequence of the 2520th to 3350th nucleotides,
a nucleotide sequence of the 3351st to 5177th nucleotides,
a nucleotide sequence of the 4485th to 4574th nucleotides,
a nucleotide sequence of the 5178th to 5918th nucleotides,
a nucleotide sequence of the 5544th to 5633rd nucleotides,
a nucleotide sequence of the 5919th to 6371st nucleotides and
a nucleotide sequence of the 6372nd to 9362nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or comprising a nucleotide sequence obtained by substituting at least one nucleotide of said nucleotide sequence in accordance with the degeneracy of the genetic code.

11. An isolated deoxyribonucleic acid consisting of the following nucleotide sequence and a complementary sequence to said nucleotide sequence:
a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or consisting of a nucleotide sequence obtained by substituting at least one nucleotide of said nucleotide sequence in accordance with the degeneracy of the genetic code.

12. An isolated antigen polypeptide consisting of an amino acid sequence coded for by a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof.

13. A method for producing a non-A, non-B hepatitis virus antigen polypeptide, which comprises:
(a) inserting a deoxyribonucleic acid into a replicable expression vector selected from a replicable plasmid vector and a replicable animal virus genome vector to obtain a replicable recombinant DNA molecule comprising said replicable plasmid vector and said deoxyribonucleic acid inserted therein when said replicable expression vector is a replicable plasmid vector, or to obtain a replicable recombinant animal virus genome molecule comprising said animal virus genome vector and said deoxyribonucleic acid inserted therein when said replicable expression vector is a replicable animal virus genome vector,
said deoxyribonucleic acid consisting of a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or consisting of a nucleotide sequence obtained by substituting at least one nucleotide of said nucleotide sequence in accordance with the degeneracy of the genetic code;
(b) transfecting cells of a microorganism or eukaryotic cell culture with said recombinant DNA molecule when said replicable expression vector used in step (a) is a replicable expression plasmid vector, to thereby form a transformant, followed by selection of said transformant from parent cells of the microorganism or eukaryotic cell culture;
(c) culturing said transformant obtained in step (b) to thereby express said deoxyribonucleic acid and produce a non-A, non-B hepatitis virus antigen peptide, or culturing said replicable recombinant animal virus genome molecule obtained in step (a) to thereby express said deoxyribonucleic acid and said animal virus genome and produce a non-A, non-B hepatitis virus antigen polypeptide and an animal virus; and
(d) isolating said non-A, non-B hepatitis virus antigen polypeptide.

14. A replicable recombinant DNA molecule comprising a replicable plasmid vector and a deoxyribonucleic acid consisting of the following nucleotide sequence and a complementary sequence to said nucleotide sequence:
a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or consisting of a nucleotide sequence obtained by substituting at least one nucleotide of said nucleotide sequence in accordance with the degeneracy of the genetic code.

15. A replicable recombinant animal virus genome molecule comprising an animal virus genome vector and a deoxyribonucleic acid consisting of the following nucleotide sequence and a complementary sequence to said nucleotide sequence:
a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or consisting of a nucleotide sequence obtained by substituting at least one nucleotide of said nucleotide sequence in accordance with the degeneracy of the genetic code.

16. A diagnostic reagent for the detection of non-A, non-B hepatitis by hybridization or polymerase chain reaction, comprising an effective amount, for the hybridization or polymerase chain reaction, of an isolated deoxyribonucleic acid consisting of the following nucleotide sequence and a complementary sequence to said nucleotide sequence:
a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or consisting of a nucleotide sequence obtained by substituting at least one nucleotide of said nucleotide sequence in accordance with the degeneracy of the genetic code.

17. A diagnostic reagent for the detection of non-A, non-B hepatitis by antigen-antibody reaction, comprising an effective amount, for the antigen-antibody reaction, of an isolated antigen polypeptide consisting of an amino acid sequence coded for by a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof.

18. A vaccine for non-A, non-B hepatitis, comprising an effective immunogenic amount of an isolated antigen polypeptide consisting of an amino acid sequence coded for by a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof; and at least one pharmaceutically acceptable carrier, diluent or excipient.

19. An isolated RNA synthesized using as a template a deoxyribonucleic acid consisting of the following nucleotide sequence and a complementary sequence to said nucleotide sequence:
a nucleotide sequence of the 333rd to 422nd nucleotides of the non-A, non-B hepatitis virus entire nucleotide sequence from the 1st to 9416th nucleotides which is the upper row sequence of the double stranded nucleotide sequence shown in Fig. 2(1) through Fig. 2(16) hereof,
or consisting of a nucleotide sequence obtained by substituting at least one nucleotide of said nucleotide sequence in accordance with the degeneracy of the genetic code.
